(19) Europäisches Patentamt
European Patent Office
Office européen des brevets

(11) **EP 4 394 784 A1**

(12) **EUROPEAN PATENT APPLICATION**

(43) Date of publication:
**03.07.2024 Bulletin 2024/27**

(21) Application number: **23220596.3**

(22) Date of filing: **28.12.2023**

(51) International Patent Classification (IPC):
**G16H 10/20** (2018.01)    **G16H 10/60** (2018.01)
**G16H 50/20** (2018.01)    **G16H 50/30** (2018.01)
**G16H 50/70** (2018.01)

(52) Cooperative Patent Classification (CPC):
**G16H 10/60; G16H 10/20;** G16H 50/20;
G16H 50/30; G16H 50/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.12.2022 US 202263478040 P**

(71) Applicant: **Tempus AI, Inc.**
**Chicago, IL 60654 (US)**

(72) Inventors:
• **MIOTTO, Riccardo**
**Chicago, 60654 (US)**

• **FERGUSON, Javid**
**Chicago, 60654 (US)**
• **HEILBRONER, Samuel**
**Chicago, 60654 (US)**
• **REDDY, Madhav Y.**
**Chicago, 60654 (US)**

(74) Representative: **Grund, Martin et al**
**Grund Intellectual Property Group**
**Patentanwälte und Solicitor PartG mbB**
**Steinsdorfstraße 2**
**80538 München (DE)**

Remarks:
Claims filed after the date of filing of the application
(Rule 68(4) EPC).

(54) **SYSTEMS AND METHODS FOR PREDICTING PATIENT RECRUITMENT AT CLINICAL SITES**

(57) Systems and methods for predicting clinical trial enrollment at clinical sites are provided. For a respective site, a method identifies a corresponding set of patient records that satisfies the criteria for a clinical trial of interest. Based on the corresponding set of patient records, the method generates a corresponding longitudinal tra-
jectory of patient eligibility for the clinical trial over a time period. Based on the corresponding longitudinal trajectory of patient eligibility for the clinical trial, the method predicts a corresponding set of expected values for enrollment in the clinical trial at the respective site.

**EP 4 394 784 A1**

**Description**

**CROSS REFERENCE TO RELATED APPLICATIONS**

This application claim priority to U.S. Provisional Patent Application No. 63/478,040, filed December 30, 2022, the content of which is hereby incorporated by reference herein, in its entirety, for all purposes.

**TECHNICAL FIELD**

The present disclosure relates to systems and methods for predicting patient recruitment at clinical trial sites based on electronic health/medical records.

**BACKGROUND**

Predictability in recruitment plays an important role in portfolio planning, trial design and site selection, and thus is critical to the success of clinical trials. Inaccurate recruitment estimates can result in protocol design considerations being too narrow to complete study on time or too wide resulting in difficulty in hitting efficacy/toxicity endpoints. Many clinical trials, especially in oncology, failed because they could not reach the minimum number of patients. See, for example, Fogel DB, Contemporary Clinical Trials Communications, 11:156-64 (2018).

When planning a clinical trial, sponsors need to identify in advance sites that are likely to receive patients eligible for the clinical trial, that will be effective in enrolling and retaining such patients, and that will run the clinical trial and follow all associated protocols. However, current practices for identifying such sites are not effective because they rely on analysis of trailing indicators for site selection, such as geography, recent patient statistics, past performance of similar clinical trials, and heuristic estimation based on previous clinical trial data.

For example, Lagor et al. Journal of Clinical Oncology 40(16 suppl.): 1558 (2022) suggest that the underrepresentation of minorities in clinical cancer trials could be improved by ranking clinical sites by the proportion of Black patients and the overall count of prostate cancer patients at the site as identified by medical claim codes. Bieganek C, et al., PLoS ONE 17(2):e0263193 (2022), describe the development of clinical trial enrollment models based on information derived from the ClinicalTrials.gov XML data, the U.S. Census Bureau, and an institution-specific impact score from the peer-reviewed journal Nature. Similarly, Liu J., et al., A Machine Learning Approach for Recruitment Prediction in Clinical Trial Design, arXiv:2111.07407 (2021) describe the development of clinical trial enrollment models based on historical clinical trial enrollment data.

**SUMMARY**

Given the above background, what is needed in the art are improved systems and methods for estimating patient recruitment at clinical sites. Specifically, methods and systems that better capture the current patient population at a clinical site and scale to provide future estimates of patient populations are needed. In some embodiments, the present disclosure addresses these and other needs by providing systems and methods that estimate the expected future patient population at clinical sites based on longitudinal trajectories of patient histories. In some embodiments, the present disclosure addresses these and other needs by providing systems and methods that use unstructured clinical information from electronic health/medical records ("EHRs"). These free-text clinical notes in EHRs provide more granular information about patient conditions, clinical context, diagnosis, treatment, etc., than does structured data in EHRs (e.g., medical codes) relied upon by studies such as Lagor et al., supra. In some embodiments, the present disclosure provides systems and methods that estimate the expected future patient population at clinical sites based on longitudinal trajectories of patient histories, leveraging unstructured clinical information from EHRs.

Advantageously, these strategies result in larger sample sizes, more holistic patient representations, better generalizability, and more accurate recruitment estimates. In some embodiments, the disclosed systems and methods are partially or fully automated to eliminate human intervention and thus reduce human labors and/or errors, e.g., using machine learning and/or natural language processing techniques to automatically extract criteria from clinical protocols and automatically analyze electronic health/medical records. In some embodiments, the disclosed systems and methods are configured to provide other additional or optional information associated with patient recruitment, such as additionally/optionally recruitment scores (rather than simply using the population statistics as score) or additionally/optionally more accurate recruitment temporal expectations.

In some aspects, the disclosed systems and methods can be used for prediction of any number of occurrences at a clinical site, including, patient demand levels, rates of particular diagnoses, use of a particular therapy, supply use, etc. By phenotyping any predictive event relating to the patients as their records appear in the EHR, one can predict on a time period basis the number of occurrences that may appear over time in the future as well as assist monitoring for changes in the expected rate of occurrences.

One aspect of the present disclosure provides a method for predicting clinical trial enrollment. The method is performed at a computer system that includes one or more processors and memory. The method includes identifying, for each respective site in a plurality of sites, a corresponding set of patient records from a corresponding plurality of patient records for the respective site. Each respective patient record in the corresponding plurality of patient records includes corresponding data from an electronic medical record (EMR) or electronic health record (EHR) for a respective patient in a corresponding plurality of patients. The identifying includes filtering the corresponding plurality of patient records to identify the corresponding set of patient records in the corresponding plurality of patient records that each includes, for each respective criterion in a plurality of criteria for a clinical trial, an expression indicating satisfaction of the respective criterion in the corresponding data. The method also includes generating, for each respective site in the plurality of sites, a corresponding longitudinal trajectory of patient eligibility for the clinical trial over a time period at the respective site based on the corresponding set of patient records for the respective site. The method further includes predicting, for each respective site in the plurality of sites, a corresponding set of expected values for enrollment in the clinical trial at the respective site based on the corresponding longitudinal trajectory of patient eligibility for the clinical trial. Each expected value in the corresponding set of expected values is predicted for a target time period in a set of target time periods.

In some embodiments, the method includes evaluating a description of the clinical trial using language pattern recognition to determine the plurality of criteria for the clinical trial. In some embodiments, the language pattern recognition includes a machine learning (ML) model trained to identify language related to the plurality of criteria in the description of the clinical trial. In some embodiments, the language pattern recognition uses a natural language processing (NLP) technique to identify language related to the plurality of criteria in the description of the clinical trial. In some embodiments, the NLP technique includes lexical analysis, morphological analysis, syntax analysis, parsing, semantic analysis, discourse integration, pragmatic analysis, or any combination thereof.

In some embodiments, for each respective site in the plurality of sites, the corresponding data from the EMR or EHR for the respective patient in the corresponding plurality of patients includes corresponding unstructured clinical data. In some such embodiments, the filtering includes, for each respective criterion in at least a subset of the plurality of criteria, using natural language processing to identify the expression indicating satisfaction of the respective criterion in the corresponding unstructured clinical data. In some embodiments, for each respective patient record in the corresponding set of patient records, the expression indicating satisfaction of each respective criterion in at least a subset of the plurality of criteria for the clinical trial is in the corresponding unstructured clinical data.

In some embodiments, for a respective patient record in the corresponding plurality of patient records, the corresponding unstructured clinical data is obtained from a medical evaluation memorialized in the EMR or EHR for the respective patient. In some such embodiments, the plurality of medical evaluations is clustered to obtain one or more corresponding medical evaluation clusters, and the unstructured clinical data is aggregated corresponding to each medical evaluation in a respective medical evaluation cluster of the one or more corresponding medical evaluation clusters.

Another aspect of the present disclosure provides a method for predicting clinical trial enrollment. The method is performed at a computer system that includes one or more processors and memory. The method includes obtaining, for each respective site in a plurality of sites, a corresponding plurality of patient records for the respective site. Each respective patient record in the corresponding plurality of patient records includes corresponding data from an electronic medical record (EMR) or electronic health record (EHR) for a respective patient in a corresponding plurality of patients. The method also includes identifying, for each respective site in the plurality of sites and for each respective clinical trial in a set of clinical trials, a corresponding set of patient records from the corresponding plurality of patient records for the respective site. To identify a set of patient records, a corresponding plurality of criteria is extracted from the respective clinical trial. The identifying includes filtering the corresponding plurality of patient records such that each of the corresponding set of patient records includes, for each respective criterion in the corresponding plurality of criteria for the respective clinical trial, an expression indicating satisfaction of the respective criterion in the corresponding data. The method further includes generating, for each respective site in the plurality of sites and for each respective clinical trial in the set of clinical trials, a corresponding longitudinal trajectory of patient eligibility for the respective clinical trial over a corresponding time period at the respective site based on the corresponding set of patient records. In addition, the method includes predicting, for each respective site in the plurality of sites and for each respective clinical trial in the set of clinical trials, a corresponding set of expected values for enrollment in the respective clinical trial at the respective site based on the corresponding longitudinal trajectory of patient eligibility for the respective clinical trial. Each expected value in the corresponding set of expected values is predicted for a target time period in a corresponding set of target time periods.

In some embodiments, the method includes evaluating a description of the clinical trial using language pattern recognition to determine the plurality of criteria for the clinical trial. In some embodiments, the language pattern recognition includes a machine learning (ML) model trained to identify language related to the plurality of criteria in the description of the clinical trial. In some embodiments, the language pattern recognition uses a natural language processing (NLP) technique to identify language related to the plurality of criteria in the description of the clinical trial. In some embodiments, the NLP technique includes lexical analysis, morphological analysis, syntax analysis, parsing, semantic analysis, discourse integration, pragmatic analysis, or any combination thereof.

In some embodiments, for each respective site in the plurality of sites, the corresponding data from the EMR or EHR for the respective patient in the corresponding plurality of patients includes corresponding unstructured clinical data. In some such embodiments, the filtering includes, for each respective criterion in at least a subset of the plurality of criteria, using natural language processing to identify the expression indicating satisfaction of the respective criterion in the corresponding unstructured clinical data. In some embodiments, for each respective patient record in the corresponding set of patient records, the expression indicating satisfaction of each respective criterion in at least a subset of the plurality of criteria for the clinical trial is in the corresponding unstructured clinical data.

In some embodiments, for a respective patient record in the corresponding plurality of patient records, the corresponding unstructured clinical data is obtained from a medical evaluation memorialized in the EMR or EHR for the respective patient. In some such embodiments, the plurality of medical evaluations is clustered to obtain one or more corresponding medical evaluation clusters, and the unstructured clinical data is aggregated corresponding to each medical evaluation in a respective medical evaluation cluster of the one or more corresponding medical evaluation clusters.

In another aspect, the present disclosure provides a method for estimating a number of patients seeking medical attention at a clinical site. In some embodiments, the method includes evaluating a corresponding plurality of patient records including corresponding data from an electronic medical record (EMR) or electronic health record (EHR) for each respective clinical site in a set of one or more clinical sites. In some embodiments, the evaluating includes natural language processing of unstructured clinical data in the EMR/EHR. In some embodiments, the method includes generating, for the set of one or more clinical sites, a corresponding longitudinal trajectory of patients seeking medical attention over a time period at the set of one or more clinical sites based on the corresponding set of patient records. In some embodiments, the method includes predicting, for the set of one or more clinical sites, a corresponding set of expected values for patients seeking medical attention at the set of one or more clinical sites based on the corresponding longitudinal trajectory. Each expected value in the corresponding set of expected values is predicted for a target time period in a set of target time periods. In some embodiments, the estimating is for an existing clinical site. In some embodiments, the estimating is for a site without an existing clinical site. In some embodiments, the estimation for the number of patients seeking medical attention at a clinical site is for one or more clinical indications. In some embodiments, the estimation for the number of patients seeking medical attention at a clinical site is for any clinical indication.

In another aspect, the present disclosure provides a method for predicting instances of a medical diagnosis at a clinical site. In some embodiments, the method includes evaluating a corresponding plurality of patient records including corresponding data from an electronic medical record (EMR) or electronic health record (EHR) for each respective clinical site in a set of one or more clinical sites. In some embodiments, the evaluating includes natural language processing of unstructured clinical data in the EMR/EHR. In some embodiments, the method includes generating, for the set of one or more clinical sites, a corresponding longitudinal trajectory of the medical diagnosis over a time period at the set of one or more clinical sites based on the corresponding set of patient records. In some embodiments, the method includes predicting, for the set of one or more clinical sites, a corresponding set of expected values for the medical diagnosis at the set of one or more clinical sites based on the corresponding longitudinal trajectory. Each expected value in the corresponding set of expected values is predicted for a target time period in a set of target time periods. In some embodiments, the predicting is for an existing clinical site. In some embodiments, the predicting is for a site without an existing clinical site. In some embodiments, the method further comprises generating a notice when a current rate of the medical diagnosis exceeds a threshold based on the prediction for instances of the medical diagnosis over a time period. In some embodiments, the medical diagnosis is for an infection. In some embodiments, the infection is a viral infection.

In another aspect, the present disclosure provides a method for predicting instances of a prescribed or administered therapy at a clinical site. In some embodiments, the method includes evaluating a corresponding plurality of patient records including corresponding data from an electronic medical record (EMR) or electronic health record (EHR) for each respective clinical site in a set of one or more clinical sites. In some embodiments, the evaluating includes natural language processing of unstructured clinical data in the EMR/EHR. In some embodiments, the method includes generating, for the set of one or more clinical sites, a corresponding longitudinal trajectory of the prescribed or administered therapy over a time period at the set of one or more clinical sites based on the corresponding set of patient records. In some embodiments, the method includes predicting, for the set of one or more clinical sites, a corresponding set of expected values for the prescribed or administered therapy at the set of one or more clinical sites based on the corresponding longitudinal trajectory. Each expected value in the corresponding set of expected values is predicted for a target time period in a set of target time periods. In some embodiments, the predicting is for an existing clinical site. In some embodiments, the predicting is for a site without an existing clinical site. In some embodiments, the prescribed or administered therapy is for a particular therapy. In some embodiments, the prescribed or administered therapy is for a class of therapies. In some embodiments, the method further comprises generating a notice when a current rate of the prescribed or administered therapy exceeds a threshold based on the prediction for instances of the prescribed or administered therapy over a time period.

In another aspect, the present disclosure provides a method for predicting instances of supply use at a clinical site. In some embodiments, the method includes evaluating a corresponding plurality of patient records including corresponding data from an electronic medical record (EMR) or electronic health record (EHR) for each respective clinical site in a set of one or more clinical sites. In some embodiments, the evaluating includes natural language processing of unstructured clinical data in the EMR/EHR. In some embodiments, the method includes generating, for the set of one or more clinical sites, a corresponding longitudinal trajectory of the supply use over a time period at the set of one or more clinical sites based on the corresponding set of patient records. In some embodiments, the method includes predicting, for the set of one or more clinical sites, a corresponding set of expected values for the supply use at the set of one or more clinical sites based on the corresponding longitudinal trajectory. Each expected value in the corresponding set of expected values is predicted for a target time period in a set of target time periods. In some embodiments, the predicting is for an existing clinical site. In some embodiments, the predicting is for a site without an existing clinical site. In some embodiments, supply use is for a particular product. In some embodiments, the supply use is for a class of products. In some embodiments, the method further comprises generating a notice when a current rate of the supply use exceeds a threshold based on the prediction for instances of the supply use over a time period.

Another aspect of the present disclosure provides a method for predicting clinical trial enrollment of a clinical trial having a set of criteria. The method comprises, optionally, at a computer system that includes one or more processors and memory, determining a longitudinal trajectory of patient eligibility for the clinical trial by identifying, at each respective epoch in a plurality of epochs of a first time period, a respective set of patients for a first set of one or more sites by matching a corresponding plurality of clinical values extracted from an electronic medical record (EMR) or electronic health record (EHR) for each respective patient in the plurality of patients, that is valid for the respective epoch, to each criterion in the set of criteria to determine whether they satisfy each respective criterion in the set of criteria for the clinical trial during the respective epoch. The longitudinal trajectory of patient eligibility for the clinical trial over the first time period is used to extrapolate one or more expected values for the first set of one or more sites for a second time period that is other than the first time period. A first expected value for enrollment in the clinical trial over the first time period or a second time period at the first set of one or more sites is then predicted using the one or more expected values.

In some embodiments, the determining the longitudinal trajectory comprises, for a respective patient in the first plurality of patient records, determining by natural language processing the corresponding plurality of clinical values that is valid for the respective epoch for the respective patient from the EMR or EHR of the respective patient.

In some embodiments, the determining the longitudinal trajectory comprises, for a respective patient in the first plurality of patient records, determining by natural language processing at least one clinical value in the corresponding plurality of clinical values that is valid for the respective epoch for the respective patient from unstructured data in the EMR or EHR of the respective patient.

In some embodiments the natural language processing comprises string-matching, lexical analysis, morphological analysis, syntax analysis, parsing, semantic analysis, discourse integration, pragmatic analysis, or a combination thereof.

In some embodiments the determining the longitudinal trajectory queries structured clinical data in the EMR or EHR for a respective patient in the plurality of patients with a string-matching algorithm to identify a clinical value in the corresponding plurality of clinical values that is valid for an epoch in the plurality of epochs for the respective patient.

In some embodiments the determining the longitudinal trajectory applies a large language model to the EMR or EHR for a respective patient in the plurality of patients to identify a clinical value in the corresponding plurality of clinical values that is valid for an epoch in the plurality of epochs for the respective patient.

In some embodiments the plurality of patients is at least 100 patients and thus at least 100 patient records are evaluated.

In some embodiments the set of criteria comprises at least one exclusion criterion.

In some embodiments the clinical trial is for treatment of a cancer condition and the set of criteria comprises a diagnosis of the cancer condition, administration of one or more prior therapies for the cancer condition, absence or presence of one or more biomarkers, one or more demographic parameters, or any combination thereof.

In some embodiments a description of the clinical trial is evaluated using language pattern recognition to determine the set of criteria for the clinical trial.

In some embodiments the identifying further comprises, for a respective patient in the plurality of patients, clustering medical entries from the corresponding EMR or EHR by date and assigning a corresponding earliest eligibility date for the clinical trial based on a date associated with a clustered medical entry that is an earliest indication that the respective patient met the set of criteria for the clinical trial.

In some embodiments the first set of patient records spans the first time period and the longitudinal trajectory is a measure of central tendency for the number of patients identified as satisfying each respective criterion in the set of criteria in each epoch in the plurality of epochs within the first time period.

In some embodiments the measure of central tendency is a mean of the number of patients identified as satisfying each respective criterion in the set of criteria in each epoch in the plurality of epochs within the first time period.

In some embodiments the first set of patient records spans the first time period and the longitudinal trajectory is based on a trend in the number of newly eligible patients at the first set of one or more sites over a subset of the first time period.

In some embodiments an expected value in the one or more expected values is an expected number of newly eligible patients at the first set of one or more sites over the first time period.

In some embodiments an expected value in the one or more expected values is an expected number of new enrollments at the first set of one or more sites over the first time period.

In some embodiments an expected value in the one or more expected values is an expected number of new enrollments at the first set of one or more sites over the second time period.

In some embodiments the first expected value for enrollment of the clinical trial is a non-negative integer at least when an expected value in the one or more expected values is no less than 1.

In some embodiments the longitudinal trajectory forms a Poisson distribution with respect to satisfaction of the set of criteria over the plurality of epochs, wherein the Poisson distribution is characterized by a factor of the mean of the Poisson distribution.

In some embodiments the method further comprises repeating the identifying A), using B), and predicting C) for each respective additional set of one or more sites in a plurality of additional sets of one or more sites, thereby predicting the corresponding first expected value for enrollment in the clinical trial over the first time period or the second time period for each respective additional set of one or more sites.

In some embodiments the method further comprises ranking the first set of one or more sites and each respective additional set of one or more sites in the plurality of additional sets of one or more sites based on the corresponding first expected value for enrollment in the clinical trial at the corresponding site.

In some embodiments the method further comprises selecting a group of sites for the clinical trial based on comparison between the first expected value for the first set of one or more sites and the corresponding first expected value for each respective additional set of one or more sites.

In some embodiments the plurality of additional sets of one or more sites is at least 10 sets of one or more sites.

In some embodiments the first set of one or more sites is a single site and each respective set of one or more sites in the plurality of additional sets of one or more sites is a single respective site.

In some embodiments the method further comprises updating the set of criteria with one or more revised criteria for the clinical trial; and repeating, for each respective revised criteria in the one or more revised criteria, the determining A), using B), and predicting C), thereby predicting a corresponding first expected value for enrollment in the clinical trial over the first time period or the second time period for the updated set of criteria. In some such embodiments the method further comprises selecting a criterion for the clinical trial based on comparison between the first expected value for the set of first criteria and the corresponding first expected value for each respective updated set of criteria.

In some embodiments the first set of one or more sites is at least 100 sites.

In some embodiments the method further comprises opening recruitment for the clinical trial.

In some embodiments the method further comprises administering a treatment in the clinical trial to a patient.

A further aspect of the present disclosure provides a computer system for predicting clinical trial enrollment. The computer system includes one or more processors, and a non-transitory computer-readable medium including computer-executable instructions that, when executed by the one or more processors, cause the processors to perform any method of the present disclosure.

A still further aspect of the present disclosure provides a non-transitory computer-readable storage medium having stored thereon program code instructions that, when executed by a processor, cause the processor to perform any method of the present disclosure.

## BRIEF DESCRIPTION OF THE DRAWINGS

In the drawings, embodiments of the systems and method of the present disclosure are illustrated by way of example. It is to be expressly understood that the description and drawings are only for the purpose of illustration and as an aid to understanding, and are not intended as a definition of the limits of the systems and methods of the present disclosure. In addition, reference numbers refer to the same or equivalent parts of the present invention throughout the figure(s) of the drawings.

Figure 1 is a block diagram illustrating an exemplary system for estimating patient recruitment in accordance with some embodiments of the present disclosure.

Figure 2 is a block diagram illustrating a general framework of an exemplary method for estimating patient recruitment in accordance with some embodiments of the present disclosure.

Figures 3A, 3B and 3C collectively depict a flowchart illustrating an exemplary method for estimating patient recruitment in which optional embodiments are indicated by dashed boxes, in accordance with some embodiments of the present disclosure.

Figure 4 illustrates an exemplary dataset for estimating patient recruitment in accordance with some embodiments of the present disclosure.

Figure 5 illustrates an exemplary comprehensive clinical and molecular database for estimating patient recruitment in accordance with some embodiments of the present disclosure.

Figure 6 illustrates creation of an exemplary set of snapshots in estimating patient recruitment in accordance with some embodiments of the present disclosure.

Figure 7A illustrates an exemplary ranked list for a clinical trial of interest in accordance with some embodiments of the present disclosure.

Figure 7B illustrates an exemplary ranked list for another clinical trial of interest in accordance with some embodiments of the present disclosure.

Figures 8A and 8B collectively depict a flowchart illustrating an exemplary method for estimating patient recruitment in which optional embodiments are indicated by dashed boxes, in accordance with some embodiments of the present disclosure.

Figures 9A, 9B, 9C, 9D, 9E, and 9F collectively depict a flowchart illustrating an exemplary method for estimating patient recruitment in which optional embodiments are indicated by dashed boxes, in accordance with some embodiments of the present disclosure.

## DETAILED DESCRIPTION

The systems and methods of the present disclosure are configured to estimate patient recruitment at clinical sites. In various embodiments, the disclosed systems and methods identify, using relevant information extracted from the eligibility criteria of clinical trials, patients eligible from the overall site population (both historical and current patients). The disclosed systems and methods then analyze clinical histories of these patients and estimate, based on the clinical histories of these patients, the expected trend of similar patients to visit the site. Different from current practices, in some embodiments, all pieces of information available in the EHRs and EMRs (including unstructured data) are analyzed and/or longitudinal trajectories of patient histories (rather than simple statistics) are modeled. As such, the disclosed systems and methods provide more accurate recruitment estimate for each site. In some embodiments, the disclosed systems and methods also provide recruitment scores (rather than simply using the population statistics as scores) and/or recruitment temporal expectations (estimations of the number of eligible patients at different target time periods). In some embodiments, the disclosed systems and methods use machine learning (ML) and/or natural language processing (NLP) techniques are used to automatically extract criteria from clinical trial protocols and/or automatically analyze EHRs and EMRs.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, and components have not been described in detail so as not to unnecessarily obscure aspects of the embodiments.

The terminology used in the present disclosure is for the purpose of describing particular embodiments only and is not intended to be limiting of the invention. As used in the description of the invention and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes" and/or "including," when used in this specification, specify the presence of stated features, integers, steps, operations, elements, and/or components, but do not preclude the presence or addition of one or more other features, integers, steps, operations, elements, components, and/or groups thereof.

As used herein, the term "measure of central tendency" refers to a central or representative value for a distribution of values. Non-limiting examples of measures of central tendency include an arithmetic mean, weighted mean, midrange, midhinge, trimean, geometric mean, geometric median, Winsorized mean, median, and mode of the distribution of values.

As used interchangeably herein, the term "classifier" or "model" refers to a machine learning model or algorithm.

In some embodiments, a model includes an unsupervised learning algorithm. One example of an unsupervised learning algorithm is cluster analysis. In some embodiments, a model includes supervised machine learning. Nonlimiting examples of supervised learning algorithms include, but are not limited to, logistic regression, neural networks, support vector machines, Naive Bayes algorithms, nearest neighbor algorithms, random forest algorithms, decision tree algorithms, boosted trees algorithms, multinomial logistic regression algorithms, linear models, linear regression, Gradient Boosting, mixture models, hidden Markov models, Gaussian NB algorithms, linear discriminant analysis, or any combinations thereof. In some embodiments, a model is a multinomial classifier algorithm. In some embodiments, a model is a 2-stage stochastic gradient descent (SGD) model. In some embodiments, a model is a deep neural network (*e.g.,* a deep-and-wide sample-level model).

*Clustering.* In some embodiments, the model is an unsupervised clustering model. In some embodiments, the model is a supervised clustering model. Clustering algorithms suitable for use as models are described, for example, at pages 211-256 of Duda and Hart, Pattern Classification and Scene Analysis, 1973, John Wiley & Sons, Inc., New York, (hereinafter "Duda 1973") which is hereby incorporated by reference in its entirety. As an illustrative example, in some embodiments, the clustering problem is described as one of finding natural groupings in a dataset. To identify natural groupings, two issues are addressed. First, a way to measure similarity (or dissimilarity) between two samples is determined. This metric (*e.g.,* similarity measure) is used to ensure that the samples in one cluster are more like one another than they are to samples in other clusters. Second, a mechanism for partitioning the data into clusters using the similarity measure is determined. One way to begin a clustering investigation is to define a distance function and to compute the matrix of distances between all pairs of samples in the training set. If distance is a good measure of similarity, then the distance between reference entities in the same cluster is significantly less than the distance between the reference entities in different clusters. However, in some implementations, clustering does not use a distance metric. For example, in some embodiments, a nonmetric similarity function s(x, x') is used to compare two vectors x and x'. In some such embodiments, s(x, x') is a symmetric function whose value is large when x and x' are somehow "similar." Once a method for measuring "similarity" or "dissimilarity" between points in a dataset has been selected, clustering uses a criterion function that measures the clustering quality of any partition of the data. Partitions of the dataset that extremize the criterion function are used to cluster the data. Particular exemplary clustering techniques contemplated for use in the present disclosure include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering includes unsupervised clustering (*e.g.,* with no preconceived number of clusters and/or no predetermination of cluster assignments).

As used herein, the term "parameter" refers to any coefficient or, similarly, any value of an internal or external element (*e.g.,* a weight and/or a hyperparameter) in an algorithm, model, regressor, and/or classifier that can affect (*e.g.,* modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (*e.g.,* a feature) to an algorithm, model, regressor, and/or classifier. As a nonlimiting example, in some embodiments, a parameter is used to increase or decrease the influence of a node (*e.g.,* of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (*e.g.,* by error minimization and/or backpropagation methods). In some embodiments, an algorithm, model, regressor, and/or classifier of the present disclosure includes a plurality of parameters. In some embodiments, the plurality of parameters is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n \geq 600$; $n \geq 750$; $n \geq 1,000$; $n \geq 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, or $n \geq 1 \times 10^7$. As such, the algorithms, models, regressors, and/or classifiers of the present disclosure cannot be mentally performed. In some embodiments n is between 10,000 and $1 \times 10^7$, between 100,000 and $5 \times 10^6$, or between 500,000 and $1 \times 10^6$. In some embodiments, the algorithms, models, regressors, and/or classifier of the present disclosure operate in a k-dimensional space, where k is a positive integer of 5 or greater (*e.g.,* 5, 6, 7, 8, 9, 10, *etc.*). As such, the algorithms, models, regressors, and/or classifiers of the present disclosure cannot be mentally performed.

The description herein includes example systems, methods, techniques, instruction sequences, and computing machine program products that embody illustrative implementations. For purposes of explanation, numerous specific details are set forth in order to provide an understanding of various implementations of the inventive subject matter. It will be evident, however, to those skilled in the art that implementations of the inventive subject matter may be practiced without these specific details. In general, well-known instruction instances, protocols, structures, and techniques have not been shown in detail.

The description herein, for purpose of explanation, is described with reference to specific implementations. However, the illustrative discussions are not intended to be exhaustive or to limit the implementations to the precise forms disclosed. Many modifications and variations are possible in view of the disclosed teachings. The implementations are chosen and described in order to best explain the principles and their practical applications, to thereby enable others skilled in the art to best utilize the implementations and various implementations with various modifications as are suited to the particular use contemplated.

In the interest of clarity, not all of the routine features of the implementations described herein are shown and described. It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that such a design effort might be complex and time-consuming, but nevertheless be a routine undertaking of engineering for those of ordering skill in the art having the benefit of the present disclosure.

As used herein, the term "if' may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

As used herein, the term "about" or "approximately" can mean within an acceptable error range for the particular value as determined by one of ordinary skill in the art, which can depend in part on how the value is measured or determined, *e.g.,* the limitations of the measurement system. For example, "about" can mean within 1 or more than 1 standard deviation, per the practice in the art. "About" can mean a range of $\pm$ 20%, $\pm$ 10%, $\pm$ 5%, or $\pm$ 1% of a given value. Where particular values are described in the application and claims, unless otherwise stated, the term "about" means within an acceptable error range for the particular value. The term "about" can have the meaning as commonly understood by one of ordinary skill in the art. The term "about" can refer to $\pm$ 10%. The term "about" can refer to $\pm$ 5%.

Furthermore, when a reference number is given an "$i^{th}$" denotation, the reference number refers to a generic component, set, or embodiment. For instance, an element termed "element $i$" or "element-$i$" refers to the $i^{th}$ element in a set of elements or in a plurality of elements.

In addition, although the terms first, second, *etc.* may be used herein to describe various elements, these elements should not be limited by these terms. These terms are only used to distinguish one element from another. For instance, a first site could be termed a second site, and, similarly, a second site could be termed a first site, without departing from the scope of the present disclosure. The first site and the second site are both sites, but they are not the same site.

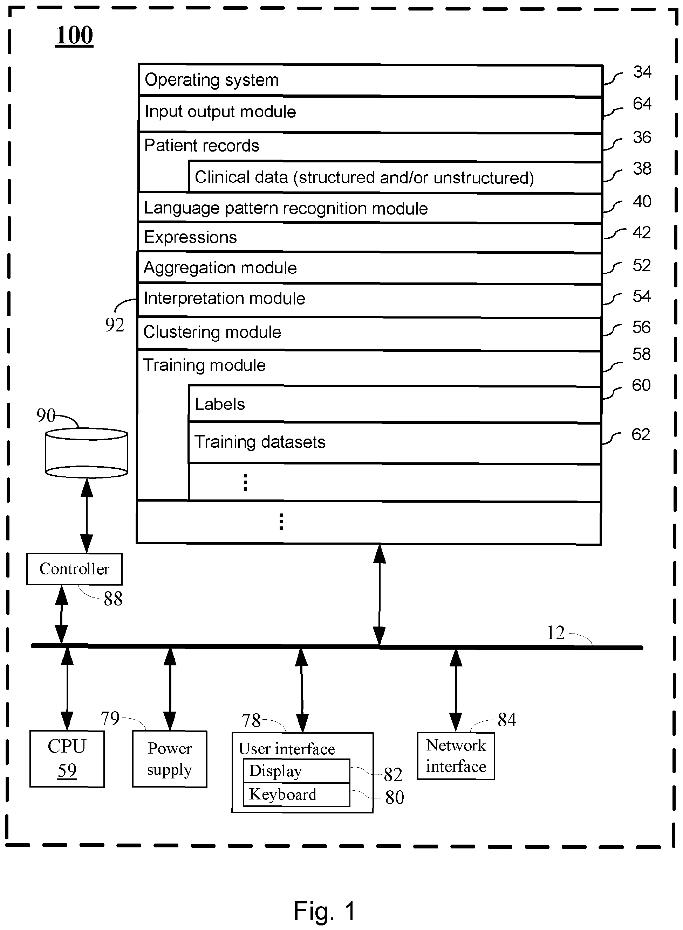

Figure 1 illustrates a computer system 100 for estimating patient recruitment at clinical sites in accordance with some embodiments of the present disclosure. In typical embodiments, the computer system 100 includes one or more computers. For purposes of illustration, the computer system 100 is shown as a single computer that includes all of the functionality of the disclosed computer system 100. However, the present disclosure is not so limited. The functionality of the computer system 100 may be spread across any number of networked computers and/or reside on each of several networked computers and/or virtual machines. One of skill in the art will appreciate that a wide array of different computer topologies are possible for the computer system 100 and all such topologies are within the scope of the present disclosure.

In some embodiments, the computer system 100 includes one or more processing units (CPUs) 59, a network or other communications interface 84, a user interface 78 (*e.g.,* including an optional display 82 and optional keyboard 80 or other form of input device), a memory 92 (*e.g.,* random access memory, persistent memory, or combination thereof), one or more magnetic disk storage and/or persistent devices 90 optionally accessed by one or more controllers 88, one or more communication busses 12 for interconnecting the aforementioned components, and a power supply 79 for powering the aforementioned components. To the extent that components of the memory 92 are not persistent, data in the memory 92 can be seamlessly shared with the non-volatile memory 90 or portions of the memory 92 that are non-volatile or persistent using known computing techniques such as caching. The memory 92 and/or memory 90 can include mass storage that is remotely located with respect to the central processing unit(s) 59. In other words, some data stored in the memory 92 and/or memory 90 may in fact be hosted on computers that are external to the computer system 100 but that can be electronically accessed by the computer system 100 over an Internet, intranet, or other form of network or electronic cable using the network interface 84. In some embodiments, the computer system 100 makes use of models that are run from the memory associated with one or more graphical processing units in order to improve the speed and performance of the system. In some alternative embodiments, the computer system 100 makes use of models that are run from the memory 92 rather than memory associated with a graphical processing unit.

In some embodiments, the memory 92 of the computer system 100 stores:

- an operating system 34 that includes procedures for handling various basic system services;
- an optional input output module 64 for obtaining, in electronic form, patient records that include structured data, unstructured data or a combination of structured and unstructured data from one or more electronic medical records (EMR) or electronic health records (EHR) for patients. In some embodiments, the input output module 64 creates a presentation with the outcome of the predicted clinical trial enrollment. In some embodiments, the input output module 64 trains a model to predict an outcome of the clinical trial enrollment using the patient records;
- patient records 36 that include clinical data for patients;
- clinical data 38 that include data from an electronic medical record (EMR) and/or electronic health record (EHR) for each patient. EHRs and EMRs typically have structured data (e.g., medical codes used by the healthcare provider for billing purposes), unstructured data (e.g., digitized records from clinical notes and observations made by physicians, physician assistants, nurses, and others while attending to the patient), or a combination of structured and unstructured data. As such, EHRs and EMRs hold a tremendous amount of clinical data that could be used to generate models for estimating patient recruitment. The EMRs and/or EHRs may be native, curated or both;
- a language pattern recognition module 40 for filtering the patient records 38 using language pattern recognition to identify patient records that include an expression related to a clinical trial criterion or a clinical condition. In some embodiments, the language pattern recognition module 40 matches one or more regular expressions against unstructured clinical data. In some embodiments, the language pattern recognition is trained by a machine learning model;
- expressions 42 that may include regular expressions for use by the language pattern recognition module 40. The expressions 42 may be optional in systems that use a machine learning model for language pattern recognition;
- optionally, an aggregation module 52;
- optionally, an interpretation module 54;
- optionally, a clustering module 56 for clustering medical evaluations memorialized in an EMR or EHR for a patient to obtain medical evaluation clusters. The clustering module 56 also aggregates unstructured clinical data corresponding to each medical evaluation in a respective medical evaluation cluster. In some embodiments, the clustering uses temporal based clustering (e.g., based on the dates of the medical evaluations memorialized in the EMR or HER). In some embodiments, the clustering is one-dimensional clustering; and
- optionally, a training module 58 that includes labels 60 and a training dataset 62.

In some implementations, one or more of the above identified data elements or modules of the computer system 100 are stored in one or more of the previously mentioned memory devices, and correspond to a set of instructions for performing a function described above. The above identified data, modules or programs (e.g., sets of instructions) need not be implemented as separate software programs, procedures or modules, and thus various subsets of these modules may be combined or otherwise re-arranged in various implementations. In some implementations, the memory 92 and/or memory 90 optionally stores a subset of the modules and data structures identified above. Furthermore, in some embodiments the memory 92 and/or memory 90 stores additional modules and data structures not described above.

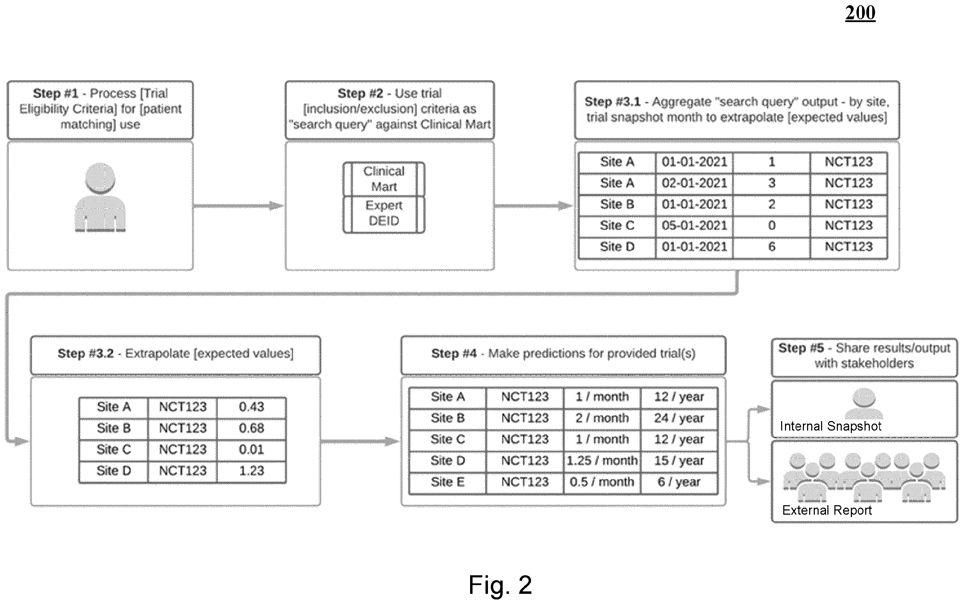

Figure 2 is a block diagram illustrating a general framework of an exemplary method 200 for estimating patient recruitment at clinical sites in accordance with some embodiments of the present disclosure.

At Step #1, a clinical trial of interest and its eligibility criteria (e.g., the clinical trial's Inclusion/Exclusion) are analyzed to extract relevant medical concepts for identifying eligible patients. In the oncology domain, the relevant medical concepts commonly include primary cancer diagnosis, therapies, biomarkers and demographic. The relevant medical concepts for the clinical trial can be extracted manually, automatically, or hybridly. For instance, in some embodiments, the relevant medical concepts are manually extracted when the clinical trial is added to the network. In some embodiments, the relevant medical concepts are automatically extracted using the language pattern recognition module 40. In some embodiments, the language pattern recognition module uses a natural language processing (NLP) technique to automatically parse the clinical trial protocol.

At Step #2, the relevant medical concepts extracted from the clinical trial eligibility criteria are used to identify patients potentially eligible for the clinical trial. In some embodiments, a set of structured query language (SQL) queries is created based on the extracted relevant medical concepts, and run against a dataset of patient records to identify those that match the eligibility criteria. Examples of SQL queries include but are not limited to patient identification, diagnosis date, name of the institution (e.g., clinical site) name, canonical name of marker, canonical name of variant type, canonical name of clinical significance, canonical name of categorical result, canonical name of gene structure type, canonical name of medication concept, number of gene structure, mutation effect, gene, cancel type, or any combination thereof. The set of SQL queries can be created manually, automatically or hybridly. In some embodiments, queries are combined into a single query and run against the dataset to identify patient records that match the eligibility criteria. In some embodiments, the patient records include electronic medical records (EMRs) and/or electronic health records (EHRs) of patients across many institutions. In some embodiments, key-word search is performed across unstructured data in identifying the patient records that match the eligibility criteria.

At Step #3.1, the identified patient records are organized by encounter to build longitudinal clinical trajectories, and the longitudinal clinical trajectories are then used to determine if and when patients are eligible for any clinical trial.

At Step #3.2, patient trial eligibility-over-time data output (*i.e.,* the output from Step #3.1) is used to derive an expected rate of eligible patient matches per site per trial, and the expected rate is then used to determine an expected value or an expected score. As used herein, the term "expected value" refers to a predicted number of patients potentially eligible for a clinical trial of interest at a site over a target time period, and the term "expected score" refers to a probability measuring how likely a site is to encounter the number of patients that match a clinical trial of interest). In some embodiments, the patient trial eligibility-over-time data output is assumed to have a Poisson distribution.

At Step #4, scores and past trends are decomposed to predict how many eligible patients a site is likely to have in different temporal snapshots, *e.g.,* how many eligible patients a site is likely to have over each target time period in a plurality of target time periods. For instance, in an embodiment, the method predicts the number of eligible patients a site is likely to have over a first target time period (*e.g.,* 1 month), over a second target time period (*e.g.,* 3 months) and over a third target time period (*e.g.,* 12 months).

At Step #5, the predicted estimates per site, clinical trial of interest and/or time snapshot(s) are provided to a user (*e.g.,* pharmaceutical company, hospital), for instance, upon a request from the user.

While Figure 2 illustrates the framework including step #1, step #2, step #3.1, step #3.2, step #4 and step #5, it should be noted that this is by way of example and it is non-limiting. Some of the illustrated steps (*e.g.,* Step #1, #Step 4, Step #5) are optional or additional, and a method of the present disclosure can but do not have to include all these steps. For instance, in some embodiments where criteria for a clinical trial are known (*e.g.,* a clinical trial that has been paused), the method can but do not have to perform Step #1 to extract the criteria. In some embodiments where the number of eligible patients in different temporal snapshots are not needed, the method can but do not have to perform Step #4 to predict the number of eligible patients in different temporal snapshots. Similarly, in some embodiments where a presentation or report is not needed, the method can but do not have to perform Step #5 to provide the predicted estimate(s) and/or time snapshots.

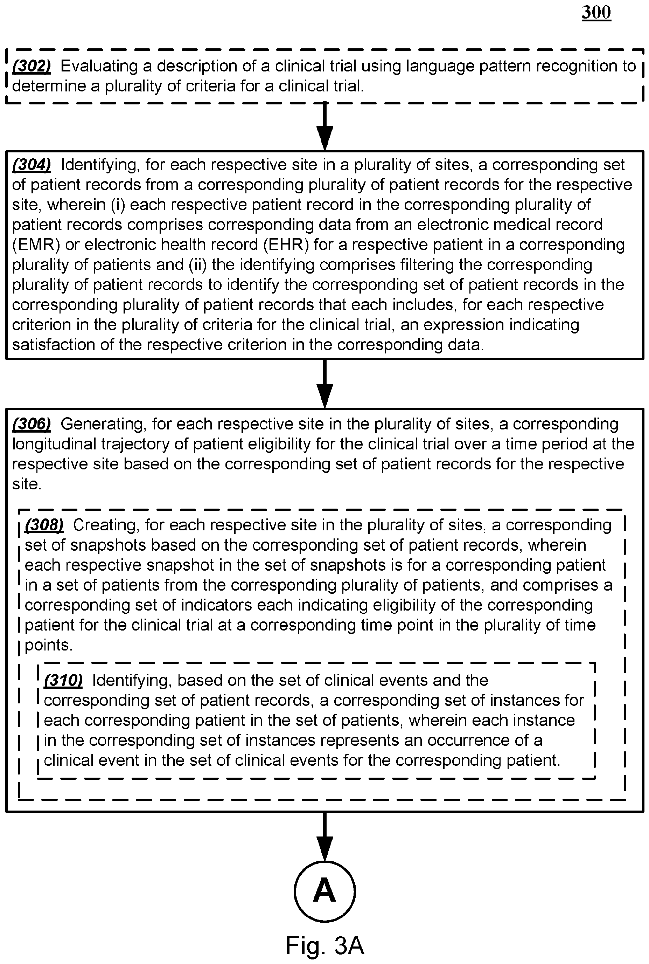

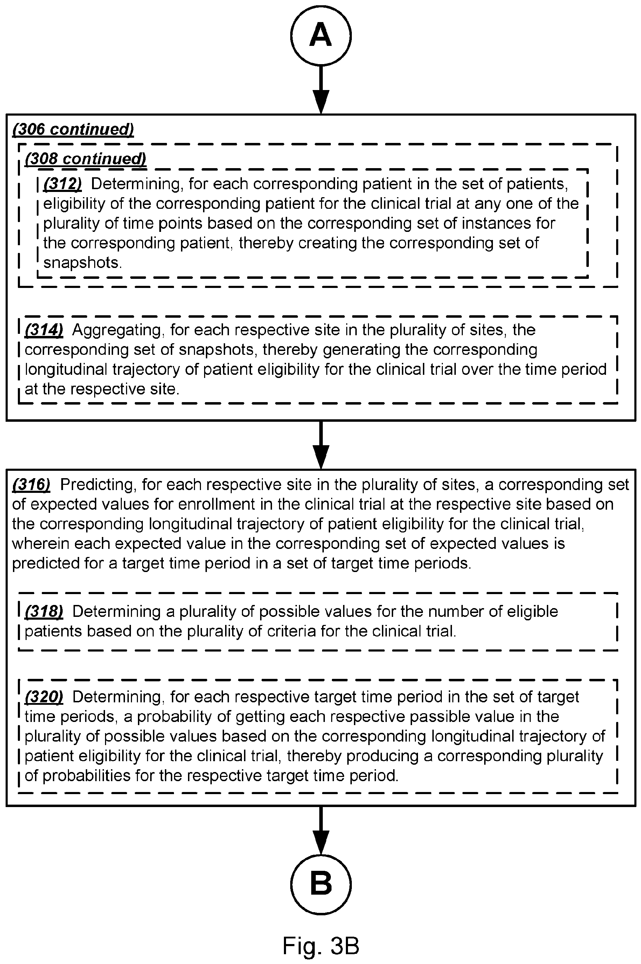

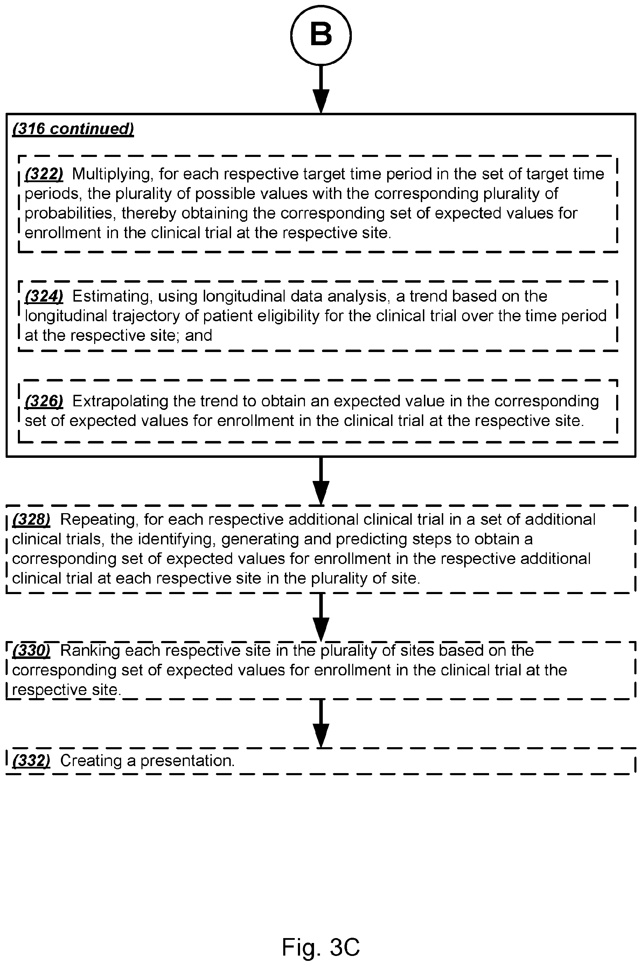

Figures 3A-3C collectively depict a flowchart illustrating an exemplary method 300 for predicting clinical trial enrollment in accordance with some embodiments of the present disclosure. In the flowchart, the preferred parts of the method are shown in solid line boxes, whereas additional, optional, or alterative parts of the method are shown in dashed line boxes. The method 300 generally includes identifying patient records that satisfy clinical trial criteria, generating longitudinal trajectories of patient eligibility based on the identified patient records and predicting expected value(s) based on the longitudinal trajectories. In some embodiments, the method includes additional, optional or alternative steps. The method is performed at a computer system, such as the computer system 100 disclosed herein.

Referring to block 302, in some embodiments, the method 300 includes evaluating a description of a clinical trial using language pattern recognition to determine a plurality of criteria for the clinical trial. The description of a critical trial to be evaluated includes but is not limited to types of patients who may enter the clinical trial, schedules of tests and procedures, drugs involved, dosages of the drugs, length of the clinical trial, purposes of the clinical trial, inclusion/exclusion criteria, ethics, or any combination thereof. In some embodiments, the description of a critical trial to be evaluated includes all the information described in the clinical trial protocol. In some embodiments, the description of a critical trial to be evaluated includes information described in one or more prior or similar clinical trials.

The language pattern recognition may include statistical pattern recognition, syntactic pattern recognition, neural pattern recognition, template-matching pattern recognition, hybrid pattern recognition, or any combination thereof. In some embodiments, the language pattern recognition includes a machine learning (ML) model trained to identify language related to the plurality of criteria in the description of the clinical trial. The ML model may be supervised (*e.g.,* linear discriminant analysis, decision trees, neural networks, or other classification algorithms) or unsupervised (*e.g.,* deep learning methods, k-means clustering, hierarchical clustering, correlation clustering, kernel principal component analysis, or other clustering algorithms).

In some embodiments, the language pattern recognition uses a natural language processing (NLP) technique to identify language related to the plurality of criteria in the description of the clinical trial. The NLP technique makes it possible for automated interpretation of the description of the clinical trial and/or automated generation of the plurality of criteria. For instance, in some embodiments, the NLP technique includes lexical analysis, morphological analysis, syntax analysis, parsing, semantic analysis, discourse integration, pragmatic analysis, or any combination thereof. The NLP automatically parses the clinical trial protocol in determining the plurality of criteria. The parsing of a clinical trial protocol can be dependency parsing, constituency parsing, or both. Dependency parsing focuses on the relationships between words in a sentence (marking things like primary objects and predicates), whereas constituency parsing focuses on building out the parse tree using a probabilistic context-free grammar.

In some embodiments, the plurality of criteria is or includes a plurality of relevant medical concepts extracted from the clinical trial or similar trials. In some embodiments, the plurality of criteria or the plurality of relevant medical concepts includes clinical events that may be grouped or hierarchized. For instance, in an embodiment, the plurality of relevant medical concepts includes one or more hierarchies of the following clinical events: CLIA, CT, GENEPANELS, GTR, ICDO3M, ICDO3T, NCI, RXNORM, UMLS, or a combination thereof.

In some embodiments, the plurality of criteria includes one or more inclusion criteria, one or more exclusion criteria, or a combination thereof. In some exemplary embodiments, the plurality criteria includes at least one exclusion criteria. In some embodiments, the clinical trial of interest is related to a cancer, and the plurality of criteria includes a primary cancer diagnosis, one or more therapies, one or more biomarkers, one or more demographic parameters, or any combination thereof.

Referring to block 304, the method 300 includes identifying, for each respective site in a plurality of sites, a corresponding set of patient records from a corresponding plurality of patient records for the respective site. Each respective patient record in the corresponding plurality of patient records includes corresponding data from an electronic medical record (EMR) or electronic health record (EHR) for a respective patient in a corresponding plurality of patients. The identifying includes filtering the corresponding plurality of patient records to identify the corresponding set of patient records in the corresponding plurality of patient records that each includes, for each respective criterion in a plurality of criteria for a clinical trial, an expression indicating satisfaction of the respective criterion in the corresponding data.

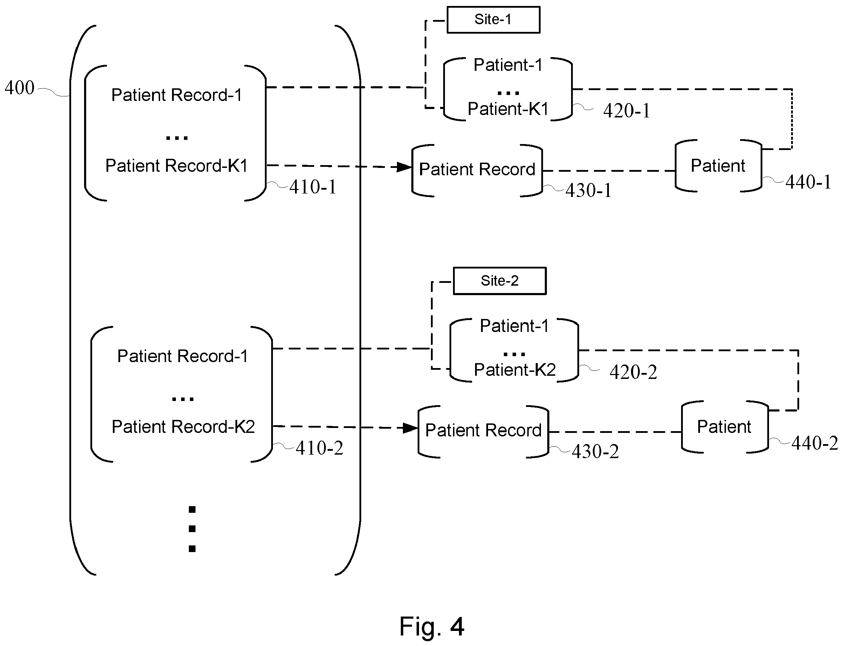

As a non-limiting example, Figure 4 illustrates a dataset 400 including patient records 36 across many institutions (*e.g.,* hospitals, clinics). In the dataset, there are a plurality of patient records for each respective site in a plurality of sites (*e.g.,* an institution under consideration for the clinical trial of interest). For instance, there is a plurality of patient records 410-1 (*e.g.,* K1 patient records, where K1 $\geq$ 2) for site-1 and there is a plurality of patient records 410-2 (*e.g.,* K2 patient records, where K2 $\geq$ 2) for site-2. The number of patient records for a site (*e.g.,* K1 for site-1) may or may not be the same as the number of patient records for another site (*e.g.,* K2 for site-2).

Where Figure 4 illustrates two sites, it should be noted that this is by way of example and is non-limiting. The dataset can include patient records for more than 2, more than 3, more than 4, more than 5, more than 10, more than 20, more than 30, more than 40, more than 50, more than 60, more than 70, more than 80, more than 90, more than 100, more than 200, more than 300, more than 400, more than 500, or more than 1000 sites. In some embodiments, the dataset includes patient records for at least 25, at least 50, at least 100, at least 200, at least 300, at least 400, at least 500, at least 1000, at least 2000, at least 3000, at least 4000 or at least 5000 sites.

Each patient record in the plurality of patient records for a site includes corresponding data from an electronic medical record (EMR) or electronic health record (EHR) for a patient in a plurality of patients. For instance, patient record-1 in the plurality of patient records 410-1 for site-1 includes an EMR or EHR for patient-1 in the plurality of patients 420-1, and patient record-K1 in the plurality of patient records 410-1 for site-1 includes an EMR or EHR for patient-K1 in the plurality of patients 420-1. Similarly, patient record-1 of the plurality of patient records 410-2 for site-2 includes an EMR or EHR for patient-1 in the plurality of patients 420-2, and patient record-K2 of the plurality of patient records 410-2 for site-1 includes an EMR or EHR for patient-K2 in the plurality of patients 420-2.

For each site, the method filters the plurality of patient records for that site to identify a set of patient records that satisfies each criterion for the clinical trial of interest. For instance, for site-1, the method filters the plurality of patient records 410-1 and identifies a set of patient records 430-1 that satisfies each criterion for the clinical trial of interest. The set of patient records 430-1 corresponds to a set of patients 440-1 for site-1. Similarly, for site-2, the method filters the plurality of patient records 410-2 and identifies a set of patient records 430-2 that satisfies each criterion for the clinical trial of interest. The set of patient records 430-2 corresponds to a set of patients 440-2 for site-2.

The set of patient records for a site that satisfies each criterion for the clinical trial of interest may be an empty set (*i.e.,* no patient record satisfies each criterion for the clinical trial of interest for that site), may be the same as the plurality of patient records for that site (*i.e.,* each and every patient record satisfies each criterion for the clinical trial of interest for that site), or may be anywhere in between. For instance, in some embodiments, no patient record in the plurality of patient records 410-1 for site-1 satisfies each criterion for the clinical trial of interest, resulting in an empty set of patient records 430-1. In some embodiments, each and every patient record in the plurality of patient records 410-1 for site-1 satisfies each criterion for the clinical trial of interest, resulting in the set of patient records 430-1 being the same as the plurality of patient records 410-1. In some embodiments, at least one record in the plurality of patient records 410-1 for site-1 satisfies each criterion for the clinical trial of interest whereas at least one record in the plurality of patient records 410-1 for site-1 does not satisfy each criterion for the clinical trial of interest, resulting in an non-empty set of patient records 430-1 with less than K1 patient records.

The dataset can include other data, such as patient records for one or more institutions that are not under consideration for the clinical trial of interest. In some embodiments, to protect patient privacy, the dataset is a de-identified dataset.

In some embodiments, the dataset is a comprehensive clinical and molecular database that includes not only standard clinical data elements but also molecular data elements.

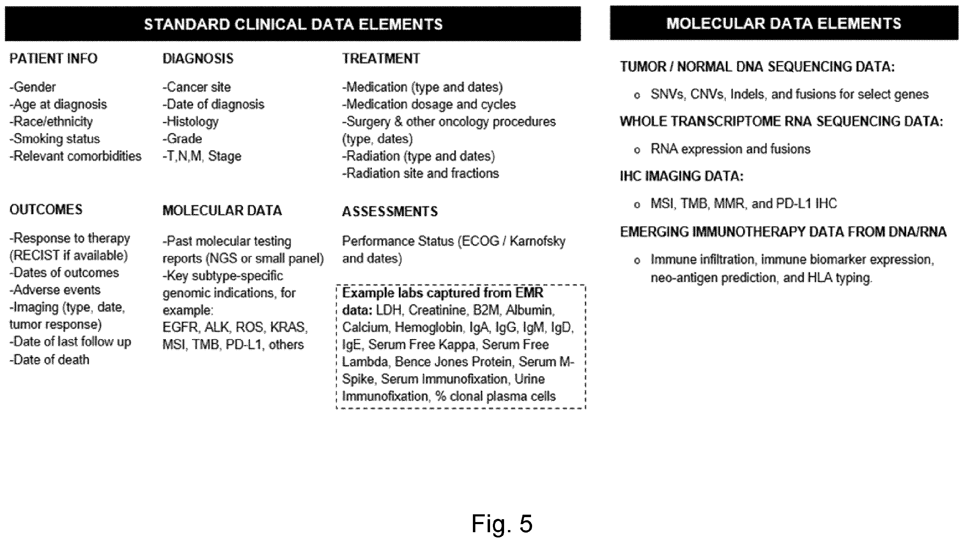

Figure 5 illustrates a non-limiting example of such a comprehensive clinical and molecular database, in which the standard clinical data elements include patient information, diagnosis, treatment, outcomes, molecular data and assessments and the molecular data elements include DNA sequencing, RNA sequencing, IHC imaging and immunotherapy data. It should be noted that a comprehensive clinical and molecular database can but do not have to include all the standard clinical data elements or all the molecular data elements listed in Figure 5. A comprehensive clinical and molecular database can also include other additional, optional or alternative elements that are not listed in Figure 5. In addition, it should be noted that a patient record for an individual patient can but do not have to include information associated with all the standard clinical data elements or all the molecular data elements listed in Figure 5. A patient record for an individual patient can also include information associated with other additional, optional or alternative elements that are not listed in Figure 5.

In some embodiments, patient records are filtered by directly evaluating each record. For example, in some embodiments, a set of patient records for a clinical site is filtered by evaluating each patient record individually, e.g., using by natural language processing, for expressions that satisfy each search criterion for a clinical trial.

In some embodiments, patient records are evaluated prior to filtering. For example, in some embodiments, a set of patient records for a clinical site are evaluated to identify phenotypic information from each record, e.g., information about patient visits, medical diagnoses, treatments, prescriptions, etc. In some embodiments, the phenotypic information from each patient record is aggregated in a database, e.g., a relational database, which may or may not be site specific. In some embodiments, this evaluation is performed prior to initiating analysis for a particular clinical trial. For example, in some embodiments, information extracted from the set of patient records is used to populate a relational database, which can later be analyzed when filtering patient records to identify instances where a patient would have been eligible for a particular clinical trial.

Non-limiting examples of the type of phenotypic information that is extracted from the patient records and stored in a relational database includes patient identification, diagnosis date, name of the institution (*e.g.,* clinical site) name, canonical name of marker, canonical name of variant type, canonical name of clinical significance, canonical name of categorical result, canonical name of gene structure type, canonical name of medication concept, number of gene structure, mutation effect, gene, cancer type, or any combination thereof.

In some embodiments, a set of queries based on criteria for a clinical trial are developed, e.g., using structured query language (SQL), and run against all or a portion of a relational database storing information extracted from patient records from one or more clinical sites. In some embodiments, the set of queries is generated manually, e.g., through manual inspection of a description for the clinical trial. In some embodiments, the set of queries is generated automatically, e.g., through natural language processing of a description for the clinical trial. In some embodiments, the set of queries is generated through a combination of automated and manual steps. For example, in some embodiments, a set of queries is generated automatically and then reviewed by a medical professional to confirm or modify the set of queries.

In some embodiments, some or all the queries for a clinical trial of interest are combined into a single query and run against the dataset to identify patient records that match the eligibility criteria. That is, the matching is not conducted on an individual Inclusion/Exclusion (IE) basis, but rather on all the IE for that given trial. This would produce the match results at the level-of-granularity of a trial cohort.

In some embodiments, for each respective site in the plurality of sites, the data from the EMR or EHR for the respective patient in the plurality of patients includes corresponding unstructured clinical data. For instance, for site-1, the data from the EMR or EHR for patient-1 in the plurality of patients 420-1 includes corresponding unstructured clinical data, and the data from the EMR or EHR for patient-K1 in the plurality of patients 420-1 includes corresponding unstructured clinical data. Similarly, for site-2, the data from the EMR or EHR for patient-1 in the plurality of patients 420-2 includes corresponding unstructured clinical data, and the data from the EMR or EHR for patient-K2 in the plurality of patients 420-2 includes corresponding unstructured clinical data.

In some embodiments, for a respective patient record in the corresponding plurality of patient records (*e.g.,* patient record-1 in the plurality of patient records 410-1), the corresponding unstructured clinical data is obtained from a medical evaluation memorialized in the EMR or EHR for the respective patient. In some embodiments, a plurality of medical evaluations (*e.g.,* all or a portion of medical evaluations memorialized in the EMR or EHR for the respective patient record) is clustered to obtain one or more corresponding medical evaluation clusters, and the unstructured clinical data is aggregated corresponding to each medical evaluation in a respective medical evaluation cluster of the one or more corresponding medical evaluation clusters. In some embodiments, the method uses the clustering module 56 to cluster medical evaluations memorialized in an EMR or EHR for a patient and/or aggregate unstructured clinical data corresponding to each medical evaluation in a respective medical evaluation cluster. In some embodiments, the clustering is, at least in part, temporal based clustering (*e.g.,* clustering based on the dates of medical evaluations memorialized in an EMR or EHR). In some embodiments, the clustering is one-dimensional clustering. Various clustering methods, such as kernel density estimation (KDE), sliding window, and machine learning, may be used.

In some embodiments, to identify the corresponding set of patient records for a site (*e.g.,* the set of patient records 430-1 for site-1), the method filters the corresponding plurality of patient records for that site (*e.g.,* the plurality of patient records 410-1) using language pattern recognition, such as that disclosed herein with respect to the evaluation of the clinical trial to determine the plurality of criteria for the clinical trial. Additional information regarding language pattern recognition can also be found in U.S. Provisional Patent Application No. 63/420,466, which is hereby incorporated by reference in its entirety.

In some embodiments, the language pattern recognition includes, for each respective criteria in the plurality of criteria for the clinical trial, matching one or more corresponding regular expressions against the corresponding unstructured clinical data, thereby identifying the corresponding set of patient records. In some embodiments, key-word search is performed across unstructured data in identifying the patient records that match the eligibility criteria.

In some embodiments, for at least one patient record in the corresponding set of patient records, the expression indicating satisfaction of each respective criterion in at least a subset of the plurality of criteria for the clinical trial is in the corresponding unstructured clinical data. In some embodiments, each respective patient record in the corresponding set of patient records, the expression indicating satisfaction of each respective criterion in at least a subset of the plurality of criteria for the clinical trial is in the corresponding unstructured clinical data. For instance, in some embodiments, for site-1, the corresponding set of patient records 430-1 includes patient record-1 and patient record-2 from the plurality of patient records 410-1, and the expression indicating satisfaction of each respective criterion in at least a subset of the plurality of criteria for the clinical trial is in the corresponding unstructured clinical data of patient record-1 or patient record-2 or each of patient record-1 and patient record-2.

Referring to block 306, the method includes generating, for each respective site in the plurality of sites, a corresponding longitudinal trajectory of patient eligibility for the clinical trial over a time period at the respective site based on the corresponding set of patient records for the respective site. Typically, the time period includes a plurality of time points, and the corresponding longitudinal trajectory of patient eligibility for the clinical trial over the time period at the respective site includes one or more values with each value indicating the number of patients eligible for the clinical trial at the respective site at a corresponding time point in the plurality of time points. Each of the one or more values is in general a non-negative integer.

For instance, as a non-limiting example, Table 1 illustrates longitudinal trajectories of patient eligibility for a clinical trial for site-1, site-2, site-3, site-4 and site-5 over a time period that includes t1, t2, t3, t4, t5, t6, and t7 time points. In the illustrated example, for site-1, one patient is found to be potentially eligible for the clinical trial at each of time points t1, t4, t5, t6 and t7, and two patients are found to be potentially eligible at each of time points t2 and t3. For site-2, no patient is found to be potentially eligible for the clinical trial at t3, one at each of time points t2 and t7, two patients at each of time points t1 and t6, and three patients at each of time points t4 and t5.

Table 1 - Examples of Longitudinal Trajectories of Patient Eligibility

| Site | t1 | t2 | t3 | t4 | t5 | t6 | t7 |
|---|---|---|---|---|---|---|---|
| Site-1 | 1 | 2 | 2 | 1 | 1 | 1 | 1 |
| Site-2 | 2 | 1 | 0 | 3 | 3 | 2 | 1 |
| Site-3 | 2 | 4 | 1 | 1 | 0 | 4 | 3 |
| Site-4 | 5 | 0 | 2 | 3 | 1 | 4 | 2 |
| Site-5 | 2 | 6 | 6 | 4 | 3 | 1 | 1 |

Each time point (*e.g.,* t1, t2) in the plurality of time points generally represents a time interval. For instance, in some embodiments, the time interval is one day, two days, three days, four days, five days, one week, two weeks, three weeks, one month, two months, three months, one half of a year, or one year. In some embodiments, the time interval is longer than one week, longer than two weeks, longer than three weeks, longer than one month, longer than two months, or longer than three months. The length of the time interval may depend, at least in part, on the application (*e.g.,* the type of the clinical trial). In some embodiments, each time interval is of a same length, e.g., each interval is a month long. In some embodiment, each time interval is a month long, e.g., a set of N time intervals represents N consecutive months. In some embodiments, each time interval is a quarter of a year (three months), e.g., a set of N time intervals represents N consecutive quarters.

While longitudinal trajectories of patient eligibility are presented in a table, it should be noted that longitudinal trajectories of patient eligibility can be presented in any suitable format, such as charts, lists or the like. Moreover, longitudinal trajectories of patient eligibility do not have to be presented. For instance, in some embodiments, the longitudinal trajectories of patient eligibility or the information associated with the longitudinal trajectories of patient eligibility are simply stored in the memory of the computer system 100.

Referring to block 308, in some embodiments, to generate the longitudinal trajectories of patient eligibility, the method includes creating, for each respective site in the plurality of sites, a corresponding set of snapshots based on the corresponding set of patient records. Each respective snapshot in the set of snapshots is for a corresponding patient in a set of patients from the corresponding plurality of patients. Each respective snapshot in the set of snapshots includes a corresponding set of indicators each indicating eligibility of the corresponding patient for the clinical trial at a corresponding time point in the plurality of time points.

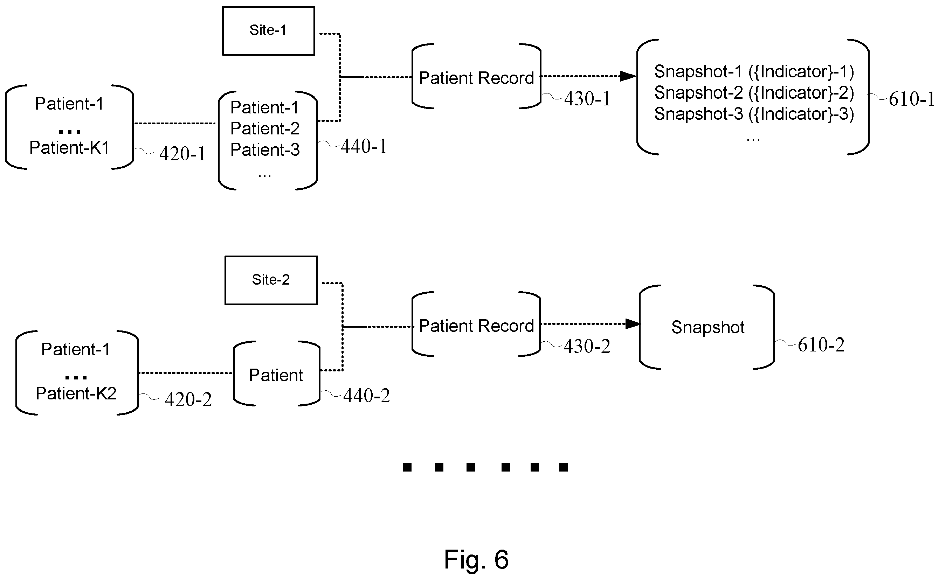

For instance, as a non-limiting example, Figure 6 illustrates that the method creates a set of snapshots 610-1 for site-1 based on the set of patient records 430-1. By way of example, Figure 6 illustrates that the set of snapshots 610-1 includes snapshot-1, snapshot-2 and snapshot-3, created respectively for patient-1, patient-2 and patient-3 in the set of patients 440-1 from the corresponding plurality of patients 420-1. The snapshot-1 in the set of snapshots 610-1 includes a set of indicators, designated by {indicator}-1, with each indicator indicating patient-1 in the set of patients 440-1 eligible for the clinical trial at a time point in the plurality of time points (*e.g.,* t1, t2, t3, t4, t5, t6 and t7). Similarly, the snapshot-2 in the set of snapshots 610-1 includes a set of indicators, designated by {indicator}-2, with each indicator indicating patient-2 in the set of patients 440-1 eligible for the clinical trial at a time point in the plurality of time points. The snapshot-3 in the set of snapshots 610-1 includes a set of indicators, designated by {indicator}-3, with each indicator indicating patient-3 in the set of patients 440-1 eligible for the clinical trial at a time point in the plurality of time points.

A snapshot may not include any indicator or include an indicator for each considered time point. By way of example, Table 2 shows the snapshot-1, snapshot-2 and snapshot-3 in the set of snapshots 610-1. The snapshot-1 includes an indicator (e.g., "x" in the table) indicating patient-1 in the set of patients 440-1 eligible for the clinical trial at t2 and an indicator indicating patient-1 in the set of patients 440-1 eligible for the clinical trial at t3. The snapshot-2 includes an indicator for each time point in the plurality of time points t1, t2, t3, t4, t5, t6 and t7, indicating patient-2 in the set of patients 440-1 eligible for the clinical trial at all considered time points. The snapshot-3 includes no indicator for any time point in the plurality of time points, indicating patient-3 in the set of patients 440-1 not eligible for the clinical trial at any considered time points. The snapshot-1, snapshot-2 and snapshot-3 collectively produces the longitudinal trajectory of patient eligibility for site-1 illustrated in Table 1, which finds one patient potentially eligible for the clinical trial at each of time points t1, t4, t5, t6 and t7, and two patients potentially eligible at each of time points t2 and t3.

Table 2 - Examples of Snapshots

| Snapshot | t1 | t2 | t3 | t4 | t5 | t6 | t7 |
|---|---|---|---|---|---|---|---|
| Snapshot-1 | | x | x | | | | |
| Snapshot-2 | x | x | x | x | x | x | x |
| Snapshot-3 | | | | | | | |

While snapshots are presented in a table, it should be noted that a snapshot can be presented in any suitable format, such as charts, lists, datasets or the like. Moreover, snapshots do not have to be presented. For instance, in some embodiments, the snapshots or the information associated with the snapshots are simply stored in the memory of the computer system 100.

In some embodiments, for at least one snapshot in the set of snapshots (e.g., snapshot-3 in the set of snapshots 610-1), the corresponding set of indicators is an empty set, indicating the corresponding patient (*e.g.,* patient-3) is not eligible for the clinical trial at any one of the plurality of time points. In some embodiments, for at least one snapshot in the set of snapshots (*e.g.,* snapshot-1 in the set of snapshots 610-1), the corresponding set of indicators includes one or more indicators, indicating that the corresponding patient (*e.g.,* patient-1) is eligible for the clinical trial at one or more time points in the plurality of time points (*e.g.,* t1, t2). In some embodiments, for at least one snapshot in the set of snapshots (*e.g.,* snapshot-2 in the set of snapshots 610-1), the corresponding set of indicators includes an indicator for each time point in the plurality of time points, indicating that the corresponding patient (patient) is eligible for the clinical trial at each of the plurality of time points (*e.g.,* t1, t2, t3, t4, t5, t6 and t7).

For an individual site (*e.g.,* site-1), the set of snapshots (*e.g.,* the set of snapshots 610-1) may contain a single snapshot or multiple snapshots depending on the number of the corresponding set of patient records (*e.g.,* the set of patient records 430-1). In some embodiments, for each respective site in at least a subset of the plurality of sites, the corresponding set of snapshots consists of a single snapshot. In some embodiments, for each respective site in at least a subset of the plurality of sites, the corresponding set of snapshots includes a plurality of snapshots (*e.g.,* 2, 3, 4, 5, more than 5, more than 10, more than 20, more than 50, more than 100, more than 500, more than 1000 snapshots).

Referring to block 310 and block 312, in some embodiments, a set of clinical events is extracted based on the plurality of criteria for the clinical trial. In such embodiments, to create a set of snapshots based on the corresponding set of patient records, the method includes (i) identifying, based on the set of clinical events and the corresponding set of patient records, a corresponding set of instances for each corresponding patient in the set of patients, wherein each instance in the corresponding set of instances represents an occurrence of a clinical event in the set of clinical events for the corresponding patient, and (ii) determining, for each corresponding patient in the set of patients, eligibility of the corresponding patient for the clinical trial at any one of the plurality of time points based on the corresponding set of instances for the corresponding patient, thereby creating the corresponding set of snapshots.

Examples of clinical events include but are not limited to CLIA, CT, GENEPANELS, GTR, ICDO3M, ICDO3T, NCI, RXNORM, UMLS, or any combination thereof. In some embodiments, the set of clinical events includes a plurality of clinical events. For instance, in some embodiments, the set of clinical events includes more than 2, more than 3, more than 4, more than 5, more than 10, more than 15, more than 20, more than 25, more than 30, more than 35, more than 40, more than 45, or more than 50 clinical events.

By way of example, suppose that the set of clinical events including clinical event-1, clinical event-2 and clinical event-3. Table 3 shows a set of identified instances for a patient (*e.g.,* patient-1 in the set of patients 440-1) based on the set of clinical events and the corresponding set of patient records (*e.g.,* the set of patient records 430-1). Each instance represents an occurrence of a clinical event for the patient. The time (*e.g.,* date, dates) at which the clinical event occurs is used to determine whether the occurrence of the clinical event falls within any time point in the plurality of time points. In the illustrated example, clinical event-1 occurs at t2 and t3, clinical event-2 occurs at t1, t2, t3, t5 and t7, and clinical event-3 occurs at t2, t3, t4, t5 and t6. In other words, all of the clinical event-1, clinical event-2 and clinical event-3 occur at t1 and t2. This produces the snapshot-1 illustrated in Table 2, which includes an indicator indicating patient-1 in the set of patients 440-1 eligible for the clinical trial at t2 and an indicator indicating patient-1 in the set of patients 440-1 eligible for the clinical trial at t3.

Table 3 - Examples of Instances

| Clinical Event | t1 | t2 | t3 | t4 | t5 | t6 | t7 |
|---|---|---|---|---|---|---|---|
| Clinical Event-1 |  | x | x |  |  |  |  |
| Clinical Event-2 | x | x | x |  | x |  | x |
| Clinical Event-3 |  | x | x | x | x | x |  |

In some embodiments, instances representing occurrences of clinical events are grouped or hierarchized. For instance, in some embodiment, instances representing occurrences of clinical events are first grouped categorically (*e.g.,* "disease", "demographic", "molecular", "medication"/"treatment") and then aggregated temporally per a unit of time (*e.g.,* per week or month or three months). In some embodiments, to enable utilization of multiple codesets that can be used across different EMRs and/or EHRs, a clinical code mapping standardization and/or integration is used to organize the instances representing occurrences of clinical events hierarchically.

In some embodiments, the eligibility of the corresponding patient for the clinical trial at any one of the plurality of time points is determined using a model trained by machine learning (*e.g.,* training module 58). Machine learning methods allow a computer system to perform automatic (e.g., through software programs) learning from a set of factual data belonging to a specific application field (e.g., domain). Given such a training set, machine learning methods are able to extract patterns and relationships from the data themselves. An extensive discussion about machine learning methods and their applications can be found in Mitchell, 1997, Machine Learning, McGraw-Hill and U.S. Patent No. 8,843,482, each of which is hereby incorporated by reference. Well-known machine learning methods include decision trees, association rules, neural networks and Bayesian methods.

Referring to block 314, in some embodiments, the method includes aggregating, for each respective site in the plurality of sites, the corresponding set of snapshots, thereby generating the corresponding longitudinal trajectory of patient eligibility for the clinical trial over the time period at the respective site. For instance, in some embodiments, for each respective site (*e.g.,* site-1) in at least a subset of the plurality of sites, the corresponding set of snapshots (the set of snapshots 610-1) includes a plurality of snapshots (*e.g.,* snapshot-1, snapshot-2 and snapshot-3). The method aggregates the plurality of snapshots (*e.g.,* snapshot-1, snapshot-2 and snapshot-3 in the set of snapshots 610-1), and thus generates a longitudinal trajectory of patient eligibility for the clinical trial for respective site (*e.g.,* the longitudinal trajectory of patient eligibility for site-1 illustrated in Table 1).

In some embodiments, the aggregating of the corresponding set of snapshots is performed using longitudinal data analysis. Examples of longitudinal analysis include but are not limited to K-means clustering, group based trajectory modeling, hierarchical cluster analysis, latent class analysis, latent class growth mixture modeling, or any combination thereof.

Referring to block 316, the method includes predicting, for each respective site in the plurality of sites, a corresponding set of expected values for enrollment in the clinical trial at the respective site based on the corresponding longitudinal trajectory of patient eligibility for the clinical trial, wherein each expected value in the corresponding set of expected values is predicted for a target time period in a set of target time periods.

As used herein, the term "expected value" refers to a predicted number of patients eligible for a clinical trial of interest at a site for a target time period. An expected value may be determined using probability and statistical analysis, models trained by machine learning, or the like.

In some embodiments, a target time period is one day, two days, three days, four days, five days, one week, two weeks, three weeks, one month, two months, one quarter of a year, one half of a year, or one year. In some embodiments, a target time period is at least one week, at least two weeks, at least three weeks, at least one month, at least two months, at least one half of a year, at least one year, or at least two years. In some embodiments, a target time period is at most one month, at most two months, at most one half of a year, at most one year, or at most two years. In some embodiments, a target time period in the set of target time periods is the same as or multiple times of a time interval (*e.g.,* t1, t2) disclosed herein with respect to the longitudinal trajectories of patient eligibility.

In some embodiments, the set of target time periods consists of a single target time period (*e.g.,* either one month or three months) and the method predicts a single expected value for each respective site in the plurality of sites.

In some embodiments, the set of target time periods includes a plurality of target time periods (*e.g.,* one month, two months, three months, one half of a year, one year). In such embodiments, the method predicts a plurality of expected values (*e.g.,* predicting an expected value for each of one month, two months, three months, one half of a year, one year) for each respective site in the plurality of sites. As such, the methods generates a temporal expectation for each respective site in the plurality of sites.

For instance, as a non-limiting example, in some embodiments, the set of target time periods includes a first target time period (*e.g.,* one month) and a second target time period (*e.g.,* one quarter of a year) that is longer than the first target time period. The method predicts a first expected value for the first target time period and a second expected value for the second target time period. The predicted expected values for the first and second target periods collectively form a temporal expectation for the respective site. As another non-limiting example, in some embodiments, the set of target time periods further includes a third target time period (*e.g.,* one half of a year or one year) that is longer than the second target time period. The method predicts a third expected value for the third target time period. The predicted expected values for the first, second and third target periods collectively form a temporal expectation for the respective site.

Referring to block 318, block 320 and block 322, in some embodiments, in predicting the corresponding set of expected values for enrollment in the clinical trial at the respective site, the method includes (i) determining a plurality of possible values for the number of eligible patients based on the plurality of criteria for the clinical trial, (ii) determining, for each respective target time period (epoch) in the set of target time periods (plurality of epochs of a first time period), a probability of getting each respective possible value in the plurality of possible values based on the corresponding longitudinal trajectory of patient eligibility for the clinical trial, thereby producing a corresponding plurality of probabilities for the respective target time period, and (iii) multiplying, for each respective target time period in the set of target time periods, the plurality of possible values with the corresponding plurality of probabilities, thereby obtaining the corresponding set of expected values for enrollment in the clinical trial at the respective site.

For instance, for a site (*e.g.,* site-1), the method determines a plurality of possible values $X(x_i, x_2, ... x_K)$ for the number of eligible patients based on the plurality of criteria for the clinical trial, where K is an integer greater than 1. The method then determines, for a respective target time period, a probability $p_k$ (k = 1, 2, ..., K) of getting each respective possible value in the plurality of possible values based on the corresponding longitudinal trajectory of patient eligibility for the clinical trial, thereby producing a corresponding plurality of probabilities $P(p_i, p_2, ... p_K)$ for the respective target time period. The method obtains the expected value for the respective target time period using Equation (1):

$$E = \sum_{k=1}^{K} x_k p_k \qquad (1)$$

In some embodiments, the plurality of possible values includes at least 2, at least 3, at least 4, at least 5, at least 6, at least 7, at least 8, at least 9, at least 10, at least 15, at least 20, at least 30, at least 40 or at least 50 possible values. In some embodiment, X is a non-negative integer set, *e.g.,* {0, 1, 2, 3, ...}, or a subset of non-negative integer set.

In some embodiments, a longitudinal trajectory of patient eligibility for a site (*e.g.,* the longitudinal trajectory of patient legibility for site-1 illustrated in Table 1) for the clinical trial of interest is assumed to have a Poisson distribution. In probability theory and statistics, the Poisson distribution is a discrete probability distribution that expresses the probability of a given number of events (*e.g.,* the number of patients eligible for a clinical trial) occurring in a fixed interval of time or space if these events occur with a known constant mean rate and independently of the time since the last event. In such embodiments, the method calculates, for each respective site, an average rate γ of the number of eligible patients by dividing the sum of all the eligible patients for the respective site by the time period (*e.g.,* the sum of t1, t2, ... t7) over which the longitudinal trajectory of patient legibility is generated. The method then calculates the expected value for each respective target time period using Equation (2), where t denotes the respective target time period:

$$E = \gamma t \qquad (2)$$

In some embodiments, $\gamma$ is calculated as the average rate of the number of eligible patients per month. In such embodiments, the expected value for a target time period of one month is $\gamma$, the expected value for a target time period of two months is $2 \times y$, and the expected value for a target time period of one year is $12 \times \gamma$.

In some embodiments, additionally or optionally, the method uses Poisson's probability mass function (PMF) to calculate a probability (e.g., expected scores) of getting a possible value (e.g., $x_1$, $x_2$) in the plurality of possible values using Equation (3), where n denotes the number of possible eligible patients (e.g., $x_1$, $x_2$):

$$E\,(n\ in\ t) = \frac{(\gamma t)^n e^{-\gamma t}}{n!} \qquad (3)$$

By way of example, Table 4 shows expected values predicted for a targeted time period (e.g., one month) for a clinical trial of interest (e.g., Ayala AL-TNBC-01 Tenacity with NCTID of NCT04461600) at various sites, and calculated probabilities of getting possible values. In Table 4, the possible values are {0, 1, 2, 3, 4, 5, 6, 7, 8, 9}. Table 4 also lists the Poisson cumulative distribution function (CDF), i.e., the probability of having a number of potentially eligible patient that is equal to or less than a possible value.

Table 4 - Examples of Predicted Expected Values and Probabilities of Getting Possible Values

| Institution | Predicted AVG (for a target time period) | Poiason PMF Probabilities | Poisson CDF Probabilities |
|---|---|---|---|
| Midwestern Regional Medical Center | 1 | {0: 0.36788, 1: 0.36788, 2: 0.18394, 3: 0.06131, 4: 0.01533, 5: 0.00307, 6: 0.00051, 7: 7e-05, 8: 1e-05, 9: 0.0} | {0: 0.36788, 1: 0.73576, 2: 0.9197, 3: 0.98101, 4: 0.99634, 5: 0.99941, 6: 0.99992, 7: 0.99999, 8: 1.0, 9: 1.0} |
| Henry Ford Allegiance Health - Jackson | 1 | {0: 0.36788, 1: 0.36788, 2: 0.18394, 3: 0.06131, 4: 0.01533, 5: 0.00307, 6: 0.00051, 7: 7e-05, 8: 1e-05, 9: 0.0} | {0: 0.36788, 1: 0.73576, 2: 0.9197, 3: 0.98101, 4: 0.99634, 5: 0.99941, 6: 0.99992, 7: 0.99999, 8: 1.0, 9: 1.0} |
| Northwestern - Developmental Therapeutics Institute | 1 | {0: 0.36788, 1: 0.36788, 2: 0.18394, 3: 0.06131, 4: 0.01533, 5: 0.00307, 6: 0.00051, 7: 7e-05, 8: 1e-05, 9: 0.0} | {0: 0.36788, 1: 0.73576, 2: 0.9197, 3: 0.98101, 4: 0.99634, 5: 0.99941, 6: 0.99992, 7: 0.99999, 8: 1.0, 9: 1.0} |
| Northwestern - Maggie Daley Women's Cancer Center | 1 | {0: 0.36788, 1: 0.36788, 2: 0.18394, 3: 0.06131, 4: 0.01533, 5: 0.00307, 6: 0.00051, 7: 7e-05, 8: 1e-05, 9: 0.0} | {0: 0.36788, 1: 0.73576, 2: 0.9197, 3: 0.98101, 4: 0.99634, 5: 0.99941, 6: 0.99992, 7: 0.99999, 8: 1.0, 9: 1.0} |
| Northwestern Lurie Cancer Center | 1 | {0: 0.36788, 1: 0.36788, 2: 0.18394, 3: 0.06131, 4: 0.01533, 5: 0.00307, 6: 0.00051, 7: 7e-05, 8: 1e-05, 9: 0.0} | {0: 0.36788, 1: 0.73576, 2: 0.9197, 3: 0.98101, 4: 0.99634, 5: 0.99941, 6: 0.99992, 7: 0.99999, 8: 1.0, 9: 1.0} |
| AO - Chicago Ridge | 0.5 | {0: 0.60653, 1: 0.30327, 2: 0.07582, 3: 0.01264, 4: 0.00158, 5: 0.00016, 6: 1e-05, 7: 0.0, 8: 0.0, 9: 0.0} | {0: 0.60653, 1: 0.9098, 2: 0.98561, 3: 0.99825, 4: 0.99983, 5: 0.99999, 6: 1.0, 7: 1.0, 8: 1.0, 9: 1.0} |
| Comprehensive Cancer Centers of Nevada - Twain | 0.5 | {0: 0.60653, 1: 0.30327, 2: 0.07582, 3: 0.01264, 4: 0.00158, 5: 0.00016, 6: 1e-05, 7: 0.0, 8: 0.0, 9: 0.0} | {0: 0.60653, 1: 0.9098, 2: 0.98561, 3: 0.99825, 4: 0.99983, 5: 0.99999, 6: 1.0, 7: 1.0, 8: 1.0, 9: 1.0} |

(continued)

| Institution | Predicted AVG (for a target time period) | Poiason PMF Probabilities | Poisson CDF Probabilities |
|---|---|---|---|
| Nebraska Cancer Specialists - Parent | 0.31579 | {0: 0.72921, 1: 0.23028, 2: 0.03636, 3: 0.00383, 4: 0.0003, 5: 2e-05, 6: 0.0, 7: 0.0, 8: 0.0, 9: 0.0} | {0: 0.72921, 1: 0.95949, 2: 0.99585, 3: 0.99968, 4: 0.99998, 5: 1.0, 6: 1.0, 7: 1.0, 8: 1.0, 9: 1.0} |
| Stockton Hematology Oncology Medical Group-Stockton Suite B | 0.27778 | {0: 0.75746, 1: 0.21041, 2: 0.02922, 3: 0.00271, 4: 0.00019, 5: 1e-05, 6: 0.0, 7: 0.0, 8: 0.0, 9: 0.0} | {0: 0.75746, 1: 0.96787, 2: 0.9971, 3: 0.9998, 4: 0.99999, 5: 1.0, 6: 1.0, 7: 1.0, 8: 1.0, 9: 1.0} |
| Oncology Consultants - Houston | 0.23077 | {0: 0.79392, 1: 0.18321, 2: 0.02114, 3: 0.00163, 4: 9e-05, 5: 0.0, 6: 0.0, 7: 0.0, 8: 0.0, 9: 0.0} | {0: 0.79392, 1: 0.97714, 2: 0.99828, 3: 0.9999, 4: 1.0, 5: 1.0, 6: 1.0, 7: 1.0, 8: 1.0, 9: 1.0} |

Referring to block 324 and block 326, alternatively, in some embodiments, in predicting corresponding set of expected values for enrollment in the clinical trial at the respective site, the method includes (i) estimating, using longitudinal data analysis, a trend based on the longitudinal trajectory of patient eligibility for the clinical trial over the time period at the respective site, and (ii) extrapolating the trend to obtain an expected value in the corresponding set of expected values for enrollment in the clinical trial at the respective site. In some embodiments, the longitudinal data analysis utilizes a growth mixture model, a latent class growth analysis, longitudinal latent class analysis, or any combination thereof, to estimate the trend.

Referring to block 328, in some embodiments, the method includes repeating, for each respective additional clinical trial in a set of additional clinical trials, the identifying, generating and predicting steps to obtain a corresponding set of expected values for enrollment in the respective additional clinical trial at each respective site in the plurality of site. In some embodiments, for at least one additional clinical trial in the set of additional clinical trials, the method also includes one or more other steps disclosed herein. For instance, in some embodiments, for at least one additional clinical trial in the set of additional clinical trials, the method includes the evaluating of the description of the clinical trial, the ranking of sites, the creating of a presentation, or any combination thereof.

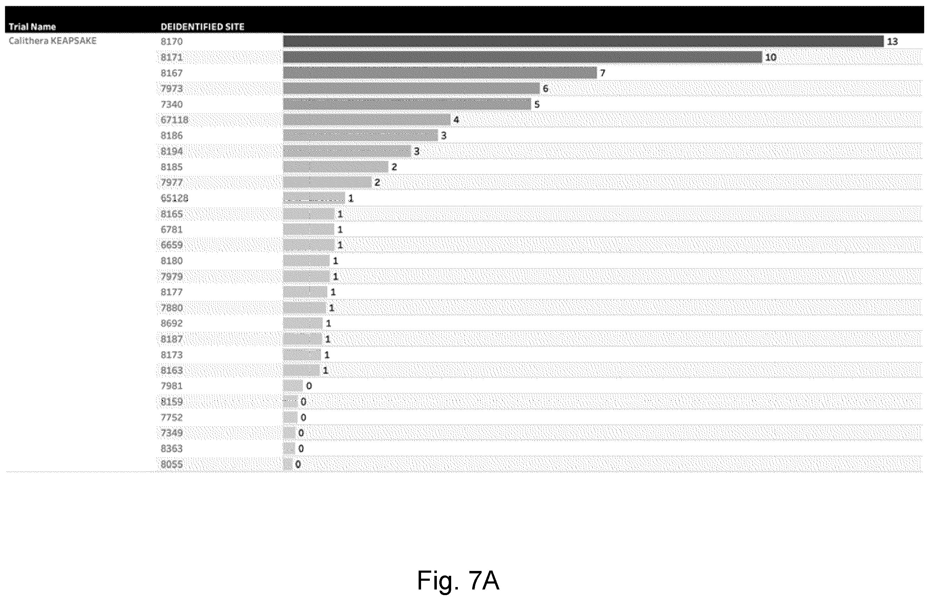

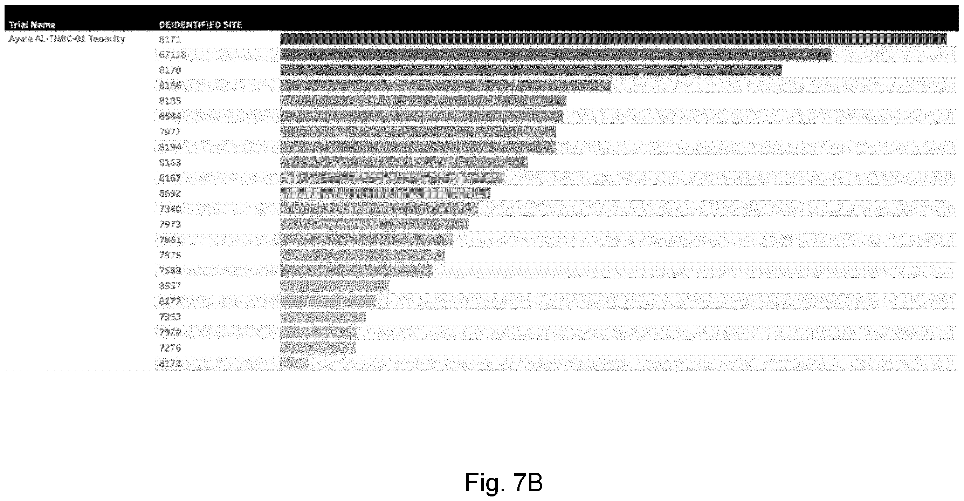

Referring to block 330, in some embodiments, the method includes ranking each respective site in the plurality of sites based on the corresponding set of expected values for enrollment in the clinical trial at the respective site. For instance, as a non-limiting example, Figure 7A illustrates a ranked list (sites are deidentified) for a clinical trial of interest. As another non-limiting example, Figure 7B illustrates a ranked list (sites are deidentified) for another clinical trial of interest.

Referring to block 332, in some embodiments, the method includes creating a presentation. In some embodiments, the presentation includes (i) for each respective site in the plurality of sites, the corresponding set of expected values for enrollment in the clinical trial at the respective site, (ii) for each respective site in the plurality of sites, the corresponding set of expected values for enrollment in at least one additional clinical trial in the set of additional clinical trials, (iii) for each respective site in the plurality of sites, the corresponding set of expected values for enrollment in each additional clinical trial in the set of additional clinical trials, (iv) the ranking of each respective site in the plurality of sites for the clinical trial, (v) the ranking of each respective site in the plurality of sites for at least one additional clinical trial in the set of additional clinical trials, (vi) the ranking of each respective site in the plurality of sites for each clinical trial in the set of additional clinical trials, or (vii) any combination thereof. In some embodiments, the presentation includes a list, a table, a chart, a diagram, a graph, or any combination thereof. In some embodiments, the creating of the presentation is in response to a request from a user (e.g., an institution who wants to conduct a clinical trial). In some embodiments, the content of the presentation is tailored to suit the user's need or preference.

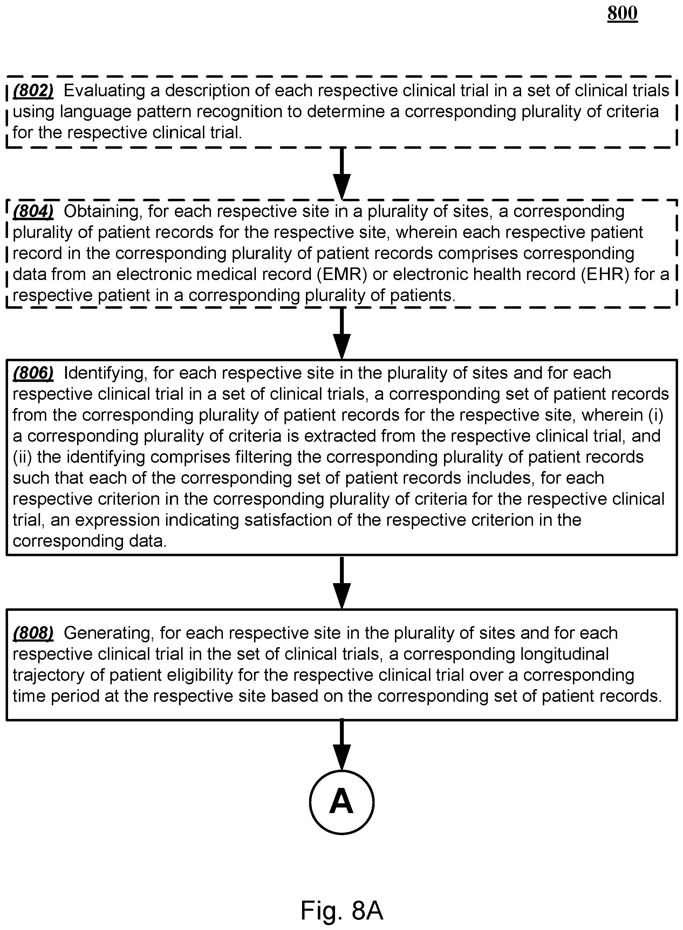

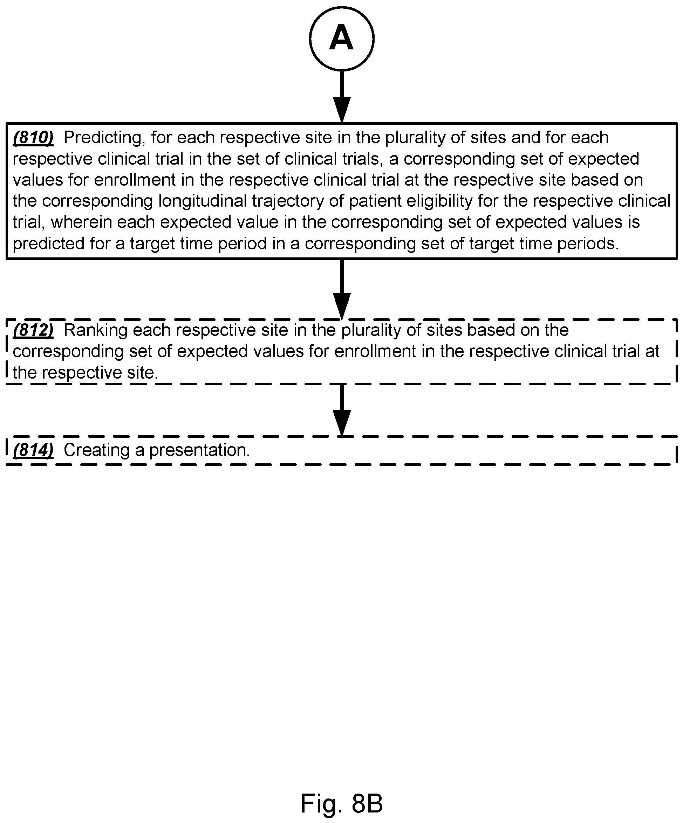

Figure 8A and 8B collectively depict a flowchart illustrating an exemplary method 800 in accordance with some embodiments of the present disclosure. In the flowchart, the preferred parts of the method are shown in solid line boxes, whereas additional, optional, or alternative parts of the method are shown in dashed line boxes. The method 800 is configured to predict clinical trial enrollment for each respective clinical trial in a set of clinical trials of interest. In some embodiments, the set of clinical trials consists of a single clinical trial. In some embodiments, the set of clinical trials includes more than 1, more than 2, more than 3, more than 4, more than 5, more than 10, more than 20, more than 30, more than 40, more than 50, more than 60, more than 70, more than 80, more than 90, or more than 100 clinical trials. The method 800 is performed at a computer system, such as the computer system 100 disclosed herein.

Referring to block 802, in some embodiments, the method 800 includes evaluating a description of each respective clinical trial in a set of clinical trials using language pattern recognition to determine a corresponding plurality of criteria for the respective clinical trial. In some embodiments, for each respective clinical trial, the evaluating step of the method 800 is the same as or similar to the evaluating step of the method 300.

Referring to block 804, in some embodiments, the method 800 includes obtaining, for each respective site in a plurality of sites, a corresponding plurality of patient records for the respective site. Each respective patient record in the corresponding plurality of patient records includes corresponding data from an electronic medical record (EMR) or electronic health record (EHR) for a respective patient in a corresponding plurality of patients. In some embodiments, at least some patient records are obtained from the computer system 100 (e.g., patient records that have been stored in the computer system 100). In some embodiments, at least some patient records are obtained from a system other than the computer system 100 (e.g., a database that is not stored in the computer system 100).

In some embodiments, the plurality of sites includes more than 2, more than 3, more than 4, more than 5, more than 10, more than 20, more than 30, more than 40, more than 50, more than 60, more than 70, more than 80, more than 90, more than 100, more than 200, more than 300, more than 400, more than 500, or more than 1000 sites. In some embodiments, the plurality of sites includes at least 25, at least 50, at least 100, at least 200, at least 300, at least 400, at least 500, at least 1000, at least 2000, at least 3000, at least 4000 or at least 5000 sites.

Referring to block 806, the method 800 includes identifying, for each respective site in the plurality of sites and for each respective clinical trial in a set of clinical trials, a corresponding set of patient records from the corresponding plurality of patient records for the respective site. A corresponding plurality of criteria is extracted from the respective clinical trial. The identifying includes filtering the corresponding plurality of patient records such that each of the corresponding set of patient records includes, for each respective criterion in the corresponding plurality of criteria for the respective clinical trial, an expression indicating satisfaction of the respective criterion in the corresponding data.

In some embodiments, for each respective site in the plurality of sites and for each respective clinical trial in the set of clinical trials, the identifying step of the method 800 is the same as or similar to the identifying step of the method 300. For instance, in some embodiments, for each respective site in the plurality of sites and for each respective clinical trial in the set of clinical trials, the corresponding data from the EMR or EHR for the respective patient in the corresponding plurality of patients includes unstructured clinical data. The identifying of the method 800 includes filtering the corresponding plurality of patient records using language pattern recognition. For at least one patient record in the corresponding set of patient records, the expression indicating satisfaction of each respective criterion in at least a subset of the plurality of criteria for the respective clinical trial is in the corresponding unstructured clinical data. In some embodiments, for each respective patient record in the corresponding set of patient records, the expression indicating satisfaction of each respective criterion in at least a subset of the plurality of criteria for the respective clinical trial is in the corresponding unstructured clinical data.

In some embodiments, for at least one site (e.g., site-1) in the plurality of sites and for at least one clinical trial (e.g., clinical trial-1) in the set of clinical trials, the corresponding set of patient records is an empty set (e.g., no patient record matches the criteria for clinical trial-1 indicating no patient eligible for clinical trial-1 at site-1). In some embodiments, for at least one site (e.g., site-2) in the plurality of sites and for at least one clinical trial (e.g., clinical trial-1) in the set of clinical trials, the corresponding set of patient records includes at least one patient record (e.g., at least one patient record matches the criteria for clinical trial-1 indicating at least one patient may be eligible for clinical trial-1 at site-2). In some embodiments, for at least one site (e.g., site-3) in the plurality of sites and for at least one clinical trial (e.g., clinical trial-1) in the set of clinical trials, the corresponding set of patient records includes multiple patient records (e.g., more than one patient record matches the criteria for clinical trial-1 indicating more than one patient may be eligible for clinical trial-1 at site-3).

Referring to block 808, the method includes generating, for each respective site in the plurality of sites and for each respective clinical trial in the set of clinical trials, a corresponding longitudinal trajectory of patient eligibility for the respective clinical trial over a corresponding time period at the respective site based on the corresponding set of patient records. In some embodiments, for each respective site in the plurality of sites and for each respective clinical trial in the set of clinical trials, the generating step of the method 800 is the same as or similar to the generating step of the method 300.

Referring to block 810, the method 800 includes predicting, for each respective site in the plurality of sites and for each respective clinical trial in the set of clinical trials, a corresponding set of expected values for enrollment in the respective clinical trial at the respective site based on the corresponding longitudinal trajectory of patient eligibility for the respective clinical trial, wherein each expected value in the corresponding set of expected values is predicted for a target time period in a corresponding set of target time periods.

In some embodiments, for each respective site in the plurality of sites and for each respective clinical trial in the set of clinical trials, the predicting step of the method 800 is the same as or similar to the predicting step of the method 300.

In some embodiments, for at least one site (e.g., site-1) in the plurality of sites and for at least one clinical trial (e.g., clinical trial-1) in the set of clinical trials, the corresponding set of target time periods includes more than 1, more than 2, more than 3, more than 4, more than 5, more than 6, more than 7, more than 8, more than 9, or more than 10 target time periods. Accordingly, the corresponding set of expected values includes more than 1, more than 2, more than 3, more than 4, more than 5, more than 6, more than 7, more than 8, more than 9, or more than 10 expected values. Each expected value is predicted for a corresponding target time period in the set of target time periods. The expected values collectively produces a temporal expectation for the at least one site *(e.g.,* site-1) and for the at least one clinical trial *(e.g.,* clinical trial-1).

In some embodiments, for each respective site in the plurality of sites and for each respective clinical trial in the set of clinical trials, the corresponding set of target time periods includes more than 1, more than 2, more than 3, more than 4, more than 5, more than 6, more than 7, more than 8, more than 9, or more than 10 target time periods. Accordingly, the corresponding set of expected values includes more than 1, more than 2, more than 3, more than 4, more than 5, more than 6, more than 7, more than 8, more than 9, or more than 10 expected values. Each expected value is predicted for a corresponding target time period in the set of target time periods. The expected values collectively produces a temporal expectation for each respective site in the plurality of sites and for each respective clinical trial in the set of clinical trials.

In some embodiments, for each respective site in the plurality of sites and for each respective clinical trial in the set of clinical trials the method 800 calculates one or more expected scores, the same as or similar to those disclosed herein with respect to the method 300.

Referring to block 812, in some embodiments, the method 800 includes ranking each respective site in the plurality of sites based on the corresponding set of expected values for enrollment in the respective clinical trial at the respective site. In some embodiments, for each respective site in the plurality of sites and for each respective clinical trial in the set of clinical trials, the ranking step of the method 800 is the same as or similar to the ranking step of the method 300.

Referring to block 814, in some embodiments, the method 800 includes creating a presentation. In some embodiments, the creating of a presentation of the method 800 is the same as or similar to the creating step of the method 300. In some embodiments, the creating of a presentation of the method 800 includes the ranking of each respective site in the plurality of sites for each clinical trial in the set of clinical trials.

In some embodiments, the method (*e.g.,* method 200, 300, 800, and/or 900) disclosed herein is validated. The validation is done against a list of patients and dates on which they passed review for specific trial eligibility.

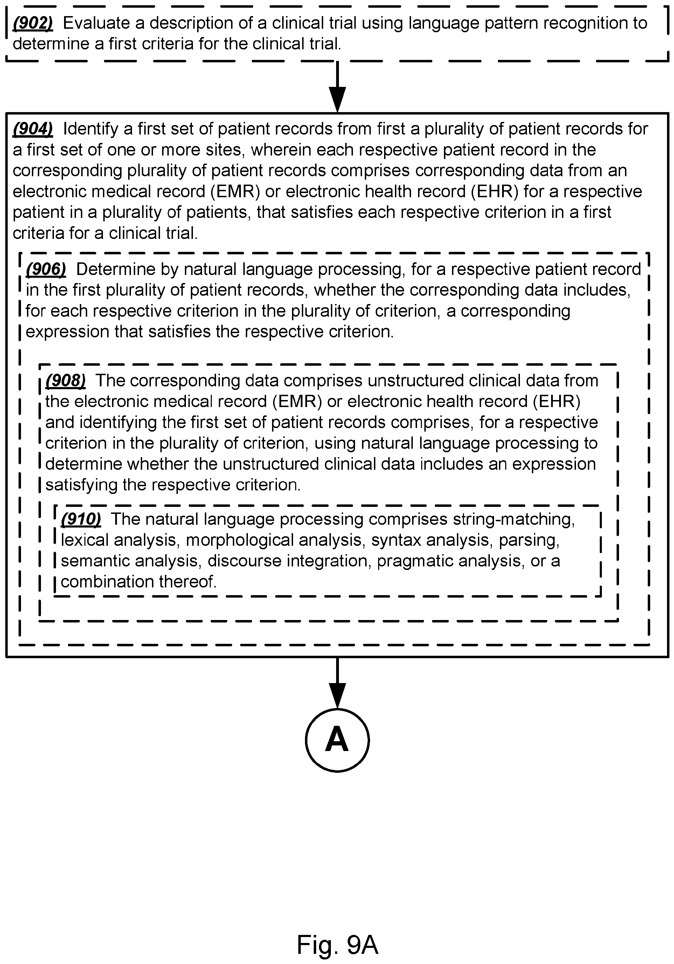

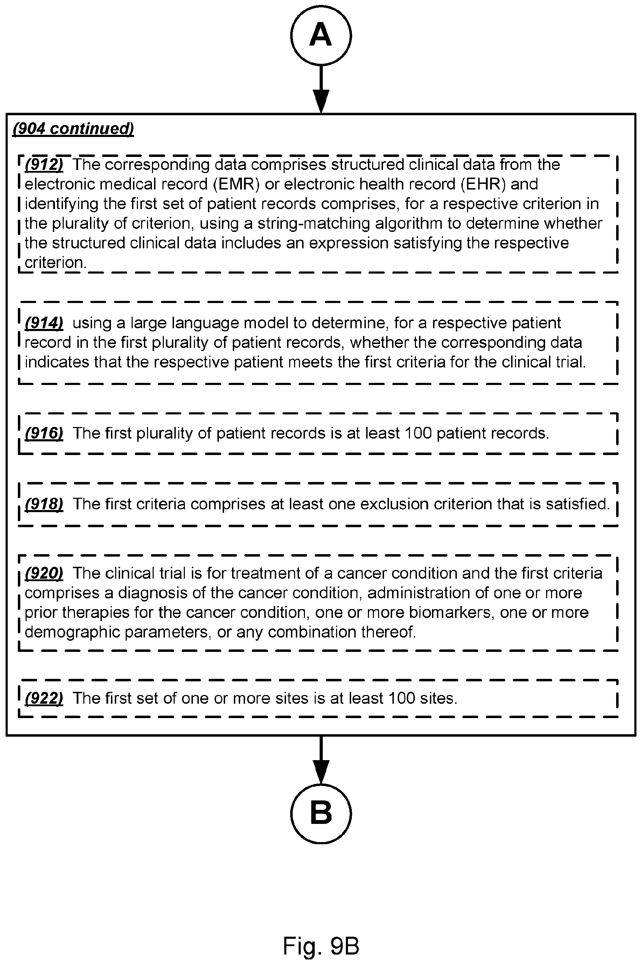

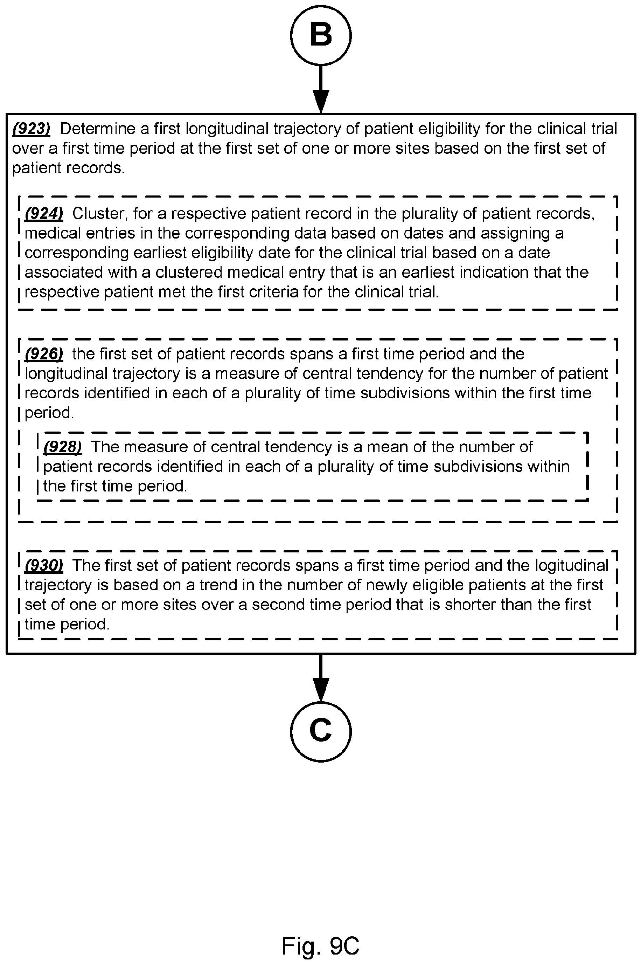

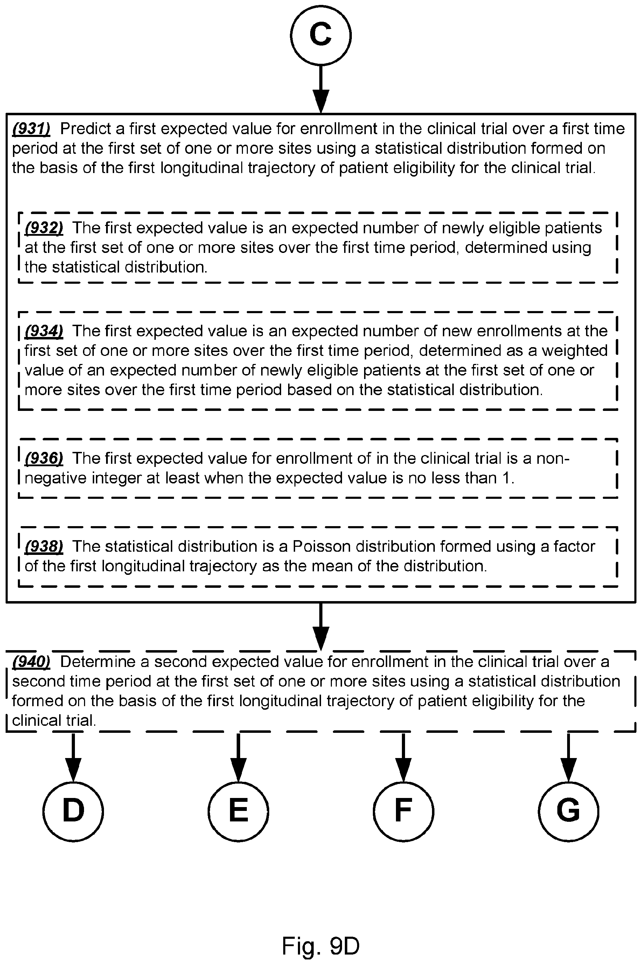

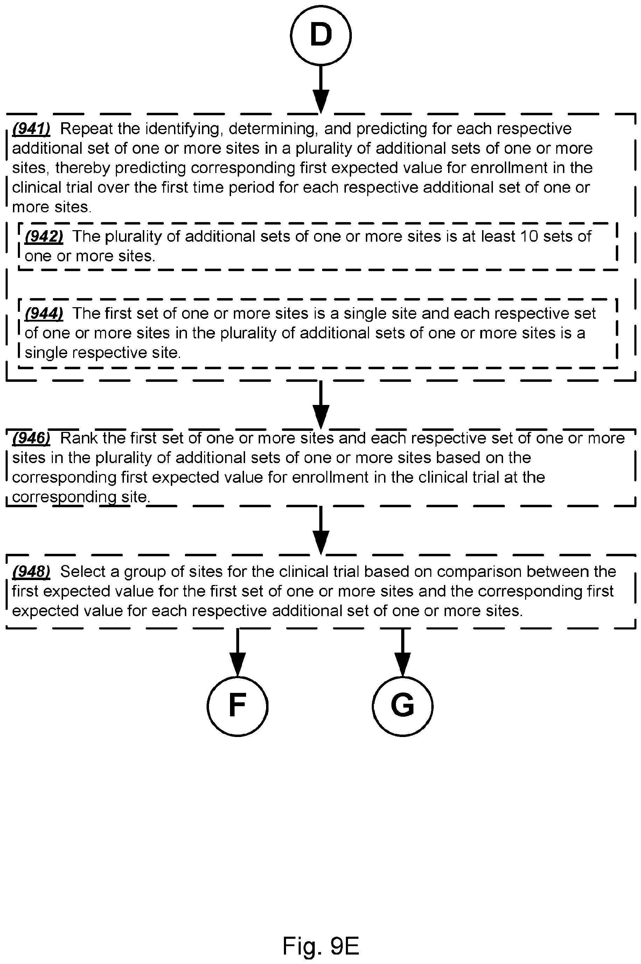

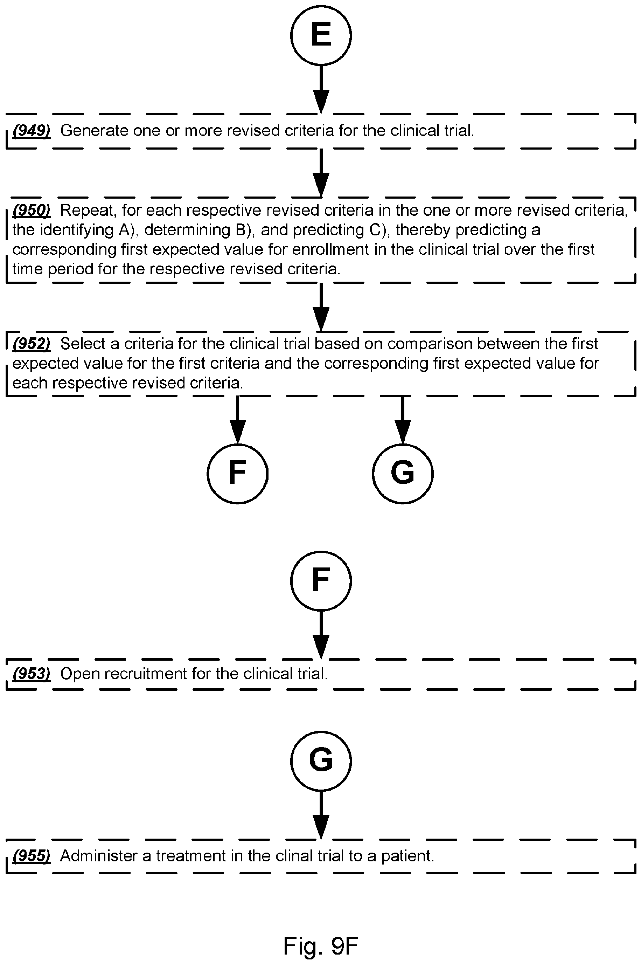

Figures 9A-9FC collectively depict a flowchart illustrating an exemplary method 900 for predicting clinical trial enrollment in accordance with some embodiments of the present disclosure. In the flowchart, the preferred parts of the method are shown in solid line boxes, whereas additional, optional, or alterative parts of the method are shown in dashed line boxes. The method 900 generally includes identifying patient records that satisfy clinical trial criteria, generating one or more longitudinal trajectories of patient eligibility based on the identified patient records, and predicting expected value(s) based on the one or more longitudinal trajectories. In some embodiments, the method includes additional, optional or alternative steps. The method is performed at a computer system, such as the computer system 100 disclosed herein.

Referring to block 902, in some embodiments, the method includes evaluating a description of a clinical trial using language pattern recognition to determine a first set of criteria for the clinical trial. In other embodiments, clinical trial criteria is pre-curated e.g., by an entity sponsoring the clinical trial or an analyst.

The language pattern recognition may include statistical pattern recognition, syntactic pattern recognition, neural pattern recognition, template-matching pattern recognition, hybrid pattern recognition, or any combination thereof. In some embodiments, the language pattern recognition includes a machine learning (ML) model trained to identify language related to the plurality of criteria in the description of the clinical trial. The ML model may be supervised (*e.g.,* linear discriminant analysis, decision trees, neural networks, or other classification algorithms) or unsupervised (*e.g.,* deep learning methods, k-means clustering, hierarchical clustering, correlation clustering, kernel principal component analysis, or other clustering algorithms). In some embodiments, the clinical trial criteria is included in a semi-structured fashion, e.g., within a participation criteria section of a submission, or draft thereof, to a regulatory body or government agency.

In some embodiments, the language pattern recognition uses a natural language processing (NLP) technique to identify language related to the plurality of criteria in the description of the clinical trial. The NLP technique makes it possible for automated interpretation of the description of the clinical trial and/or automated generation of the plurality of criteria. For instance, in some embodiments, the NLP technique includes lexical analysis, morphological analysis, syntax analysis, parsing, semantic analysis, discourse integration, pragmatic analysis, or any combination thereof. The NLP automatically parses the clinical trial protocol in determining the plurality of criteria. The parsing of a clinical trial protocol can be dependency parsing, constituency parsing, or both. Dependency parsing focuses on the relationships between words in a sentence (marking things like primary objects and predicates), whereas constituency parsing focuses on building out the parse tree using a probabilistic context-free grammar.

In some embodiments, a large language model (LLM) is used to evaluate the description of the clinical trial to identify inclusion and exclusion criteria. LLMs are transformer-based generative machine learning models trained to understand natural language. See, for example, Vaswani, Ashish, et al., "Attention is all you need," Advances in neural information processing systems 30 (2017), the content of which is incorporated herein by reference, in its entirety. For example, in some embodiments, an LLM is provided with a description of the clinical trial and prompted to list all criteria that need to be satisfied for inclusion of a patient in the clinical trial.

Referring to block 904, in some embodiments, the method includes identifying a first set of patient records from first a plurality of patient records for a first set of one or more sites, wherein each respective patient record in the corresponding plurality of patient records comprises corresponding data from an electronic medical record (EMR) or electronic health record (EHR) for a respective patient in a plurality of patients, that satisfies each respective criterion in a set of first criteria for a clinical trial.

In some embodiments, natural language processing is used to evaluate the EMR or EHR. Referring to block 906, in some embodiments, the method includes determining by natural language processing, for a respective patient record in the first plurality of patient records, whether the corresponding data includes, for each respective criterion in the plurality of criterion, a corresponding expression that satisfies the respective criterion.

Referring to block 908, in some embodiments, the corresponding data comprises unstructured clinical data from the electronic medical record (EMR) or electronic health record (EHR) and identifying the first set of patient records comprises, for a respective criterion in the plurality of criterion, using natural language processing to determine whether the unstructured clinical data includes an expression satisfying the respective criterion.

Referring to block 910, in some embodiments, the natural language processing comprises string-matching, lexical analysis, morphological analysis, syntax analysis, parsing, semantic analysis, discourse integration, pragmatic analysis, or a combination thereof.

Referring to block 912, in some embodiments, the corresponding data comprises structured clinical data from the electronic medical record (EMR) or electronic health record (EHR) and identifying the first set of patient records comprises, for a respective criterion in the plurality of criterion, using a string-matching algorithm to determine whether the structured clinical data includes an expression satisfying the respective criterion. For example, in some embodiments, one or more EMR or EHR include medical billing codes that can be parsed using one or more string-matching quieries to identify a code satisfying an inclusion criterion and/or a code indicating the presence of an exclusion criterion.

In some embodiments, characteristics relevant to some or all of the clinical trial criteria in an EMR or EHR are aggregated in a database, e.g., a referential database, containing extracted medical characteristics for one or more patients. In some embodiments, the database can be quickly queried for one or more eligibility criterion across one or more patients, including a large number of patients. In some embodiments, a set of structured query language (SQL) queries is created based on the extracted relevant medical concepts and run against a dataset of patient records to identify those that match the eligibility criteria. SQL is a programming language for storing and processing information in a relational database. However, other language pattern-matching algorithms can be used equivalently. In some embodiments, a pattern-matching algorithm (including SQL) can be used to search for terms matching any number of expressions, for example, regular expressions, Boolean expressions, exact expressions, and the like.

Referring to block 914, in some embodiments, the method includes using a large language model (LLM) to determine, for a respective patient record in the first plurality of patient records, whether the corresponding data indicates that the respective patient meets the set of first criteria for the clinical trial.

In some embodiments, the LLM comprises at least 100,000 parameters. In some embodiments, the LLM comprises at least 1 million parameters. In some embodiments, the LLM comprises at least 10 million parameters. In some embodiments, the LLM comprises at least 100 million parameters. In some embodiments, the LLM comprises at least 1 billion parameters. In some embodiments, the LLM comprises at least 10 billion parameters. In some embodiments, the LLM comprises at least 100 billion parameters. In some embodiments, the LLM comprises less than 1 trillion parameters. In some embodiments, the LLM comprises less than 100 billion parameters. In some embodiments, the LLM comprises less than 10 billion parameters. In some embodiments, the LLM comprises less than 1 billion parameters. In some embodiments, the LLM comprises from 100,000 to 100 billion parameters. In some embodiments, the LLM comprises from 1 million to 100 billion parameters. In some embodiments, the LLM comprises from 10 million to 100 billion parameters. In some embodiments, the LLM comprises from 100 million to 100 billion parameters. In some embodiments, the LLM comprises from 1 billion to 100 billion parameters. In some embodiments, the LLM comprises from 10 billion to 100 billion parameters. In some embodiments, the LLM comprises from 100,000 to 10 billion parameters. In some embodiments, the LLM comprises from 1 million to 10 billion parameters. In some embodiments, the LLM comprises from 10 million to 10 billion parameters. In some embodiments, the LLM comprises from 100 million to 10 billion parameters. In some embodiments, the LLM comprises from 1 billion to 10 billion parameters. In some embodiments, the LLM comprises from 100,000 to 1 billion parameters. In some embodiments, the LLM comprises from 1 million to 1 billion parameters. In some embodiments, the LLM comprises from 10 million to 1 billion parameters. In some embodiments, the LLM comprises from 100 million to 1 billion parameters. In some embodiments, the LLM comprises from 100,000 to 100 million parameters. In some embodiments, the LLM comprises from 1 million to 100 million parameters. In some embodiments, the LLM comprises from 10 million to 100 million parameters.

Referring to block 916, in some embodiments, the first plurality of patient records is at least 100 patient records. In some embodiments, the first plurality of patient records is at least 1000 patient records. In some embodiments, the first plurality of patient records is at least 10,000 patient records. In some embodiments, the first plurality of patient records is at least 100,000 patient records. In some embodiments, the first plurality of patient records is at least 1 million patient records. In some embodiments, the first plurality of patient records is at least 10 million patient records. In some embodiments, the first plurality of patient records is at least 100 million patient records.

In some embodiments, the first plurality of patient records is less than 10 billion patient records. In some embodiments, the first plurality of patient records is less than 1 billion patient records. In some embodiments, the first plurality of patient records is less than 100 million patient records. In some embodiments, the first plurality of patient records is less than 10 million patient records. In some embodiments, the first plurality of patient records is less than 1 million patient records. In some embodiments, the first plurality of patient records is less than 100,000 patient records.

In some embodiments, the first plurality of patient records is from 100 patient records to 10 billion patient records. In some embodiments, the first plurality of patient records is from 1000 patient records to 10 billion patient records. In some embodiments, the first plurality of patient records is from 10,000 patient records to 10 billion patient records. In some embodiments, the first plurality of patient records is from 100,000 patient records to 10 billion patient records. In some embodiments, the first plurality of patient records is from 1 million patient records to 10 billion patient records. In some embodiments, the first plurality of patient records is from 10 million patient records to 10 billion patient records. In some embodiments, the first plurality of patient records is from 100 million patient records to 10 billion patient records. In some embodiments, the first plurality of patient records is from 100 patient records to 1 billion patient records. In some embodiments, the first plurality of patient records is from 1000 patient records to 1 billion patient records. In some embodiments, the first plurality of patient records is from 10,000 patient records to 1 billion patient records. In some embodiments, the first plurality of patient records is from 100,000 patient records to 1 billion patient records. In some embodiments, the first plurality of patient records is from 1 million patient records to 1 billion patient records. In some embodiments, the first plurality of patient records is from 10 million patient records to 1 billion patient records. In some embodiments, the first plurality of patient records is from 100 million patient records to 1 billion patient records. In some embodiments, the first plurality of patient records is from 100 patient records to 100 million patient records. In some embodiments, the first plurality of patient records is from 1000 patient records to 100 million patient records. In some embodiments, the first plurality of patient records is from 10,000 patient records to 100 million patient records. In some embodiments, the first plurality of patient records is from 100,000 patient records to 100 million patient records. In some embodiments, the first plurality of patient records is from 1 million patient records to 100 million patient records. In some embodiments, the first plurality of patient records is from 10 million patient records to 100 million patient records. In some embodiments, the first plurality of patient records is from 100 patient records to 10 million patient records. In some embodiments, the first plurality of patient records is from 1000 patient records to 10 million patient records. In some embodiments, the first plurality of patient records is from 10,000 patient records to 10 million patient records. In some embodiments, the first plurality of patient records is from 100,000 patient records to 10 million patient records. In some embodiments, the first plurality of patient records is from 1 million patient records to 10 million patient records.

Referring to block 918, in some embodiments, the set of first criteria comprises at least one exclusion criterion that is satisfied. In some embodiments, a clinical trial eligibility criteria includes one or more inclusion criterion that must be present in order to admit a patient into a clinical trial. For example, in some embodiments, an inclusion criterion is a specific type of cancer, e.g., pancreatic cancer, meaning that a patient must have pancreatic cancer to be eligible for the clinical trial. In some embodiments, a clinical trial eligibility includes an exclusion criterion that cannot be present in order to admit a patient into a clinical trial. For example, in some embodiments, an exclusion criterion is a particular prior therapy, e.g., prior checkpoint inhibitor therapy, meaning that if the patient previously received checkpoint inhibitor therapy they are not eligible for the clinical trial. Generally, all inclusion criterion must be met in order for a patient to be eligible for a clinical trial, while the presence of a single exclusion criterion will exclude a patient from eligibility for the trial. However, labeling a particular criterion as either of inclusion or exclusion criterion is based on convention only, as any inclusion criterion can be turned into an exclusion criterion by excluding a patient lacking a certain condition and, vice versa, an exclusion criterion can be turned into an inclusion criterion by requiring the absence of a state or condition.

Referring to block 920, in some embodiments, the clinical trial is for treatment of a cancer condition and the set of first criteria comprises a diagnosis of the cancer condition, administration of one or more prior therapies for the cancer condition, one or more biomarkers, one or more demographic parameters, or any combination thereof.

Referring to block 922, in some embodiments, the first set of one or more sites is at least 100 sites. In some embodiments, the first set of one or more sites is a single site. In some embodiments, the first set of one or more sites is at least 10 sites. In some embodiments, the first set of one or more sites is at least 1000 sites. In some embodiments, the first set of one or more sites is at least 10,000 sites. In some embodiments, the first set of one or more sites is at least 100,000 sites. In some embodiments, the first set of one or more sites is at least 1 million sites.

In some embodiments, the first set of one or more sites is less than 10 million sites. In some embodiments, the first set of one or more sites is less than 1 million sites. In some embodiments, the first set of one or more sites is less than 100,000 sites. In some embodiments, the first set of one or more sites is less than 10,000 sites. In some embodiments, the first set of one or more sites is less than 1000 sites.

In some embodiments, the first set of one or more sites is from 1 site to 10 million sites. In some embodiments, the first set of one or more sites is from 10 sites to 10 million sites. In some embodiments, the first set of one or more sites is from 100 sites to 10 million sites. In some embodiments, the first set of one or more sites is from 1000 sites to 10 million sites. In some embodiments, the first set of one or more sites is from 10,000 sites to 10 million sites. In some embodiments, the first set of one or more sites is from 100,000 sites to 10 million sites. In some embodiments, the first set of one or more sites is from 1 million sites to 10 million sites. In some embodiments, the first set of one or more sites is from 1 site to 1 million sites. In some embodiments, the first set of one or more sites is from 10 sites to 1 million sites. In some embodiments, the first set of one or more sites is from 100 sites to 1 million sites. In some embodiments, the first set of one or more sites is from 1000 sites to 1 million sites. In some embodiments, the first set of one or more sites is from 10,000 sites to 1 million sites. In some embodiments, the first set of one or more sites is from 100,000 sites to 1 million sites. In some embodiments, the first set of one or more sites is from 1 site to 100,000 sites. In some embodiments, the first set of one or more sites is from 10 sites to 100,000 sites. In some embodiments, the first set of one or more sites is from 100 sites to 100,000 sites. In some embodiments, the first set of one or more sites is from 1000 sites to 100,000 sites. In some embodiments, the first set of one or more sites is from 10,000 sites to 100,000 sites. In some embodiments, the first set of one or more sites is from 1 site to 10,000 sites. In some embodiments, the first set of one or more sites is from 10 sites to 10,000 sites. In some embodiments, the first set of one or more sites is from 100 sites to 10,000 sites. In some embodiments, the first set of one or more sites is from 1000 sites to 10,000 sites. In some embodiments, the first set of one or more sites is from 1 site to 1000 sites. In some embodiments, the first set of one or more sites is from 10 sites to 1000 sites. In some embodiments, the first set of one or more sites is from 100 sites to 1000 sites.

Referring to block 922, in some embodiments, the method includes determining a first longitudinal trajectory of patient eligibility for the clinical trial over a first time period at the first set of one or more sites based on the first set of patient records. In some embodiments, a longitudinal trajectory of patient eligibility is expressed as a series of values for new patient eligibility over time at a set of one or more sites. For example, Table 1 illustrates longitudinal trajectories of patient eligibility for a clinical trial at sites 1-5 by providing the number of newly eligible patients identified during each of 7 time periods (t1-t7). Generally, each identified patient is assigned to a single time period, even if the medical records indicate they were eligible for the clinical trial over several evaluated periods of time. This is done to avoid overestimating the number of eligible patients for the trial at the site since a single patient can only be enrolled once in a particular clinical trial. In other embodiments, a longitudinal trajectory is expressed as a single value derived from a time series of eligibility data, e.g., derived from series data as shown in table 1.

Referring to block 924, in some embodiments, the method includes clustering, for a respective patient record in the plurality of patient records, medical entries in the corresponding data based on dates and assigning a corresponding earliest eligibility date for the clinical trial based on a date associated with a clustered medical entry that is an earliest indication that the respective patient met the set of first criteria for the clinical trial. For example, unstructured clinical data, e.g., clinical notes, are typically associated with medical examination dates in EMRs and EHRs. An assumption can be made that medical examinations occurring very close in time to each other relate to a common medical condition. For example, when admitted for in-patient surgery, evaluation, or monitoring, a patient may be examined by medical personnel over the course of several days. Rather than treating these evaluations as separate medical encounters, the dates associated with the evaluations can be used to cluster the evaluations together as they likely all relate to a common medical issue.

In some embodiments, a longitudinal trajectory is expressed as a single value, e.g., a measure of central tendency for time series eligibility data. In some embodiments, the single value is a mean of new patient eligibility time series data. For example, referring to Table 1, assuming that each time interval t is a month, the mean value for new eligible patients at site one over t1-t7 is 1.3 new eligible patients per month ([1+2+2+1+1+1+1]/7). Referring to block 926, in some embodiments, the first set of patient records spans a first time period and the longitudinal trajectory is a measure of central tendency for the number of patient records identified in each of a plurality of time subdivisions within the first time period.

Referring to block 928, in some embodiments, the measure of central tendency is a mean of the number of patient records identified in each of a plurality of time subdivisions within the first time period. Generally, any time interval can be used to derive a longitudinal trajectory that is expressed as a single value. For example, in some embodiments, the longitudinal trajectory is expressed as a measure of central tendency for the number of newly eligible patients at a site per month. In other embodiments, the longitudinal trajectory is determined for a subdivision of a day, a week, a month, two months, three months, four months, five months, six months, nine months, a year, or any other subdivision of time.

Referring to block 930, in some embodiments, the first set of patient records spans a first time period and the logitudinal trajectory is based on a trend in the number of newly eligible patients at the first set of one or more sites over a second time period that is shorter than the first time period.

Referring to block 930, in some embodiments, the method includes predicting a first expected value for enrollment in the clinical trial over a first time period at the first set of one or more sites using a statistical distribution formed on the basis of the first longitudinal trajectory of patient eligibility for the clinical trial.

Referring to block 932, in some embodiments, the first expected value is an expected number of newly eligible patients at the first set of one or more sites over the first time period, determined using the statistical distribution.

Referring to block 934, in some embodiments, the first expected value is an expected number of new enrollments at the first set of one or more sites over the first time period, determined as a weighted value of an expected number of newly eligible patients at the first set of one or more sites over the first time period based on the statistical distribution.

Referring to block 936, in some embodiments, the first expected value for enrollment of the clinical trial is a non-negative integer at least when the expected value is no less than 1.

Referring to block 938, in some embodiments, the statistical distribution is a Poisson distribution formed using a factor of the first longitudinal trajectory as the mean of the distribution.

Referring to block 940, in some embodiments, the method includes determining a second expected value for enrollment in the clinical trial over a second time period at the first set of one or more sites using a statistical distribution formed on the basis of the first longitudinal trajectory of patient eligibility for the clinical trial.

Referring to block 940, in some embodiments, the method includes determining the identifying, determining, and predicting for each respective additional set of one or more sites in a plurality of additional sets of one or more sites, thereby predicting corresponding first expected value for enrollment in the clinical trial over the first time period for each respective additional set of one or more sites.

Referring to block 942, in some embodiments, the plurality of additional sets of one or more sites is at least 10 sets of one or more sites.

Referring to block 944, in some embodiments, the first set of one or more sites is a single site and each respective set of one or more sites in the plurality of additional sets of one or more sites is a single respective site.

Referring to block 946, in some embodiments, the method includes ranking the first set of one or more sites and each respective set of one or more sites in the plurality of additional sets of one or more sites based on the corresponding first expected value for enrollment in the clinical trial at the corresponding site.

Referring to block 948, in some embodiments, the method includes selecting a group of sites for the clinical trial based on comparison between the first expected value for the first set of one or more sites and the corresponding first expected value for each respective additional set of one or more sites.

Referring to block 948, in some embodiments, the method includes generating one or more revised criteria for the clinical trial.

Referring to block 950, in some embodiments, the method includes repeating, for each respective revised criteria in the one or more revised criteria, the identifying A), determining B), and predicting C), thereby predicting a corresponding first expected value for enrollment in the clinical trial over the first time period for the respective revised criteria.

Referring to block 952, in some embodiments, the method includes selecting a criteria for the clinical trial based on comparison between the first expected value for the set of first criteria and the corresponding first expected value for each respective revised criteria.

Referring to block 952, in some embodiments, the method includes opening recruitment for the clinical trial.

Referring to block 952, in some embodiments, the method includes administering a treatment in the clinal trial to a patient.

In some embodiments, the disclosure provides a method for predicting clinical trial enrollment of a clinical trial having a set of criteria. The method is optionally performed at a computer system that includes one or more processors and memory. The method includes determining a longitudinal trajectory of patient eligibility for the clinical trial by identifying, at each respective epoch in a plurality of epochs of a first time period, a respective set of patients for a first set of one or more sites by matching a corresponding plurality of clinical values extracted from an electronic medical record (EMR) or electronic health record (EHR) for each respective patient in a plurality of patients, that is valid for the respective epoch, to each criterion in the set of criteria to determine whether they satisfy each respective criterion in the set of criteria for the clinical trial during the respective epoch. For example, as described above with reference to Table 1, example longitudinal trajectories of patient eligibility can be expressed as the number of newly eligible patients for the clinical trial within each time period in a plurality of time periods.

The method also includes using the longitudinal trajectory of patient eligibility for the clinical trial over the first time period to extrapolate one or more expected values for the first set of one or more sites for a second time period that is other than the first time period. For example, a measure of central tendency for the number of newly eligible patients across all or a subset of all periods of time evaluated. The measure of central tendency will have units of newly eligible patients over a length of time. To extrapolate this over a second time period, the measure of central tendency can be multiplied by a factor. For example, if the periods of time evaluated were each six months, the measure of central tendency can be multiplied by 1/6 to extrapolate an expected value for a one-month period of time, or by 2 to extrapolate an expected value for a year. As described herein, the expected value can also be derived based on other trends in the longitudinal trajectory. For example, where an increase or decrease over time in the number of newly eligible patients is observed, the trend can be extrapolated into the future to generate an expected value for a particular time in the future, e.g., for a two-month period starting in eight months, or a plurality of expected values for a series of time periods in the future, e.g., one for each consecutive two month periods starting in eight months and ending in thirty-two months.

The method then includes predicting, using the one or more expected values, a first expected value for enrollment in the clinical trial over the second time period at the first set of one or more sites. In some embodiments, the one or more expected values can be used to form statistical distributions, from which a most likely outcome for the number of newly eligible patients can be derived. In some embodiments, a factor or model is then applied to this most likely outcome to determine an expected value for enrollment based on the likely number of newly eligible patients, as not every newly eligible patient will likely enroll for the clinical trial.

In some embodiments, determining the longitudinal trajectory comprises, for a respective patient in the first plurality of patient records, determining by natural language processing the corresponding plurality of clinical values that is valid for the respective epoch for the respective patient from the EMR or EHR of the respective patient.

In some embodiments, determining the longitudinal trajectory comprises, for a respective patient in the first plurality of patient records, determining by natural language processing at least one clinical value in the corresponding plurality of clinical values that is valid for the respective epoch for the respective patient from unstructured data in the EMR or EHR of the respective patient.

In some embodiments, the natural language processing comprises string-matching, lexical analysis, morphological analysis, syntax analysis, parsing, semantic analysis, discourse integration, pragmatic analysis, or a combination thereof.

In some embodiments, the determining the longitudinal trajectory queries structured clinical data in the EMR or EHR for a respective patient in the plurality of patients with a string-matching algorithm to identify a clinical value in the corresponding plurality of clinical values that is valid for an epoch in the plurality of epochs for the respective patient.

In some embodiments, the corresponding data comprises structured clinical data from the electronic medical record (EMR) or electronic health record (EHR) and identifying the first set of patient records comprises, for a respective criterion in the plurality of criterion, using a string-matching algorithm to determine whether the structured clinical data includes an expression satisfying the respective criterion. For example, in some embodiments, one or more EMR or EHR include medical billing codes that can be parsed using one or more string-matching queries to identify a code satisfying an inclusion criterion and/or a code indicating the presence of an exclusion criterion.

In some embodiments, characteristics relevant to some or all of the clinical trial criteria in an EMR or EHR are aggregated in a database, e.g., a referential database, containing extracted medical characteristics for one or more patients. In some embodiments, the database can be quickly queried for one or more eligibility criterion across one or more patients, including a large number of patients. In some embodiments, a set of structured query language (SQL) queries is created based on the extracted relevant medical concepts and run against a dataset of patient records to identify those that match the eligibility criteria. SQL is a programming language for storing and processing information in a relational database. However, other language pattern-matching algorithms can be used equivalently. In some embodiments, a pattern-matching algorithm (including SQL) can be used to search for terms matching any number of expressions, for example, regular expressions, Boolean expressions, exact expressions, and the like.

In some embodiments, the determining the longitudinal trajectory applies a large language model to the EMR or EHR for a respective patient in the plurality of patients to identify a clinical value in the corresponding plurality of clinical values that is valid for an epoch in the plurality of epochs for the respective patient.

In some embodiments, the LLM comprises at least 100,000 parameters. In some embodiments, the LLM comprises at least 1 million parameters. In some embodiments, the LLM comprises at least 10 million parameters. In some embodiments, the LLM comprises at least 100 million parameters. In some embodiments, the LLM comprises at least 1 billion parameters. In some embodiments, the LLM comprises at least 10 billion parameters. In some embodiments, the LLM comprises at least 100 billion parameters. In some embodiments, the LLM comprises less than 1 trillion parameters. In some embodiments, the LLM comprises less than 100 billion parameters. In some embodiments, the LLM comprises less than 10 billion parameters. In some embodiments, the LLM comprises less than 1 billion parameters. In some embodiments, the LLM comprises from 100,000 to 100 billion parameters. In some embodiments, the LLM comprises from 1 million to 100 billion parameters. In some embodiments, the LLM comprises from 10 million to 100 billion parameters. In some embodiments, the LLM comprises from 100 million to 100 billion parameters. In some embodiments, the LLM comprises from 1 billion to 100 billion parameters. In some embodiments, the LLM comprises from 10 billion to 100 billion parameters. In some embodiments, the LLM comprises from 100,000 to 10 billion parameters. In some embodiments, the LLM comprises from 1 million to 10 billion parameters. In some embodiments, the LLM comprises from 10 million to 10 billion parameters. In some embodiments, the LLM comprises from 100 million to 10 billion parameters. In some embodiments, the LLM comprises from 1 billion to 10 billion parameters. In some embodiments, the LLM comprises from 100,000 to 1 billion parameters. In some embodiments, the LLM comprises from 1 million to 1 billion parameters. In some embodiments, the LLM comprises from 10 million to 1 billion parameters. In some embodiments, the LLM comprises from 100 million to 1 billion parameters. In some embodiments, the LLM comprises from 100,000 to 100 million parameters. In some embodiments, the LLM comprises from 1 million to 100 million parameters. In some embodiments, the LLM comprises from 10 million to 100 million parameters.

Referring to block 916, in some embodiments, the first plurality of patient records is at least 100 patient records. In some embodiments, the first plurality of patient records is at least 1000 patient records. In some embodiments, the first plurality of patient records is at least 10,000 patient records. In some embodiments, the first plurality of patient records is at least 100,000 patient records. In some embodiments, the first plurality of patient records is at least 1 million patient records. In some embodiments, the first plurality of patient records is at least 10 million patient records. In some embodiments, the first plurality of patient records is at least 100 million patient records.

In some embodiments, the first plurality of patient records is less than 10 billion patient records. In some embodiments, the first plurality of patient records is less than 1 billion patient records. In some embodiments, the first plurality of patient records is less than 100 million patient records. In some embodiments, the first plurality of patient records is less than 10 million patient records. In some embodiments, the first plurality of patient records is less than 1 million patient records. In some embodiments, the first plurality of patient records is less than 100,000 patient records.

In some embodiments, the first plurality of patient records is from 100 patient records to 10 billion patient records. In some embodiments, the first plurality of patient records is from 1000 patient records to 10 billion patient records. In some embodiments, the first plurality of patient records is from 10,000 patient records to 10 billion patient records. In some embodiments, the first plurality of patient records is from 100,000 patient records to 10 billion patient records. In some embodiments, the first plurality of patient records is from 1 million patient records to 10 billion patient records. In some embodiments, the first plurality of patient records is from 10 million patient records to 10 billion patient records. In some embodiments, the first plurality of patient records is from 100 million patient records to 10 billion patient records. In some embodiments, the first plurality of patient records is from 100 patient records to 1 billion patient records. In some embodiments, the first plurality of patient records is from 1000 patient records to 1 billion patient records. In some embodiments, the first plurality of patient records is from 10,000 patient records to 1 billion patient records. In some embodiments, the first plurality of patient records is from 100,000 patient records to 1 billion patient records. In some embodiments, the first plurality of patient records is from 1 million patient records to 1 billion patient records. In some embodiments, the first plurality of patient records is from 10 million patient records to 1 billion patient records. In some embodiments, the first plurality of patient records is from 100 million patient records to 1 billion patient records. In some embodiments, the first plurality of patient records is from 100 patient records to 100 million patient records. In some embodiments, the first plurality of patient records is from 1000 patient records to 100 million patient records. In some embodiments, the first plurality of patient records is from 10,000 patient records to 100 million patient records. In some embodiments, the first plurality of patient records is from 100,000 patient records to 100 million patient records. In some embodiments, the first plurality of patient records is from 1 million patient records to 100 million patient records. In some embodiments, the first plurality of patient records is from 10 million patient records to 100 million patient records. In some embodiments, the first plurality of patient records is from 100 patient records to 10 million patient records. In some embodiments, the first plurality of patient records is from 1000 patient records to 10 million patient records. In some embodiments, the first plurality of patient records is from 10,000 patient records to 10 million patient records. In some embodiments, the first plurality of patient records is from 100,000 patient records to 10 million patient records. In some embodiments, the first plurality of patient records is from 1 million patient records to 10 million patient records.

In some embodiments, the plurality of patients is at least 100 patients.

in some embodiments, the first set of one or more sites is at least 100 sites. In some embodiments, the first set of one or more sites is a single site. In some embodiments, the first set of one or more sites is at least 10 sites. In some embodiments, the first set of one or more sites is at least 1000 sites. In some embodiments, the first set of one or more sites is at least 10,000 sites. In some embodiments, the first set of one or more sites is at least 100,000 sites. In some embodiments, the first set of one or more sites is at least 1 million sites.

In some embodiments, the first set of one or more sites is less than 10 million sites. In some embodiments, the first set of one or more sites is less than 1 million sites. In some embodiments, the first set of one or more sites is less than 100,000 sites. In some embodiments, the first set of one or more sites is less than 10,000 sites. In some embodiments, the first set of one or more sites is less than 1000 sites.

In some embodiments, the first set of one or more sites is from 1 site to 10 million sites. In some embodiments, the first set of one or more sites is from 10 sites to 10 million sites. In some embodiments, the first set of one or more sites is from 100 sites to 10 million sites. In some embodiments, the first set of one or more sites is from 1000 sites to 10 million sites. In some embodiments, the first set of one or more sites is from 10,000 sites to 10 million sites. In some embodiments, the first set of one or more sites is from 100,000 sites to 10 million sites. In some embodiments, the first set of one or more sites is from 1 million sites to 10 million sites. In some embodiments, the first set of one or more sites is from 1 site to 1 million sites. In some embodiments, the first set of one or more sites is from 10 sites to 1 million sites. In some embodiments, the first set of one or more sites is from 100 sites to 1 million sites. In some embodiments, the first set of one or more sites is from 1000 sites to 1 million sites. In some embodiments, the first set of one or more sites is from 10,000 sites to 1 million sites. In some embodiments, the first set of one or more sites is from 100,000 sites to 1 million sites. In some embodiments, the first set of one or more sites is from 1 site to 100,000 sites. In some embodiments, the first set of one or more sites is from 10 sites to 100,000 sites. In some embodiments, the first set of one or more sites is from 100 sites to 100,000 sites. In some embodiments, the first set of one or more sites is from 1000 sites to 100,000 sites. In some embodiments, the first set of one or more sites is from 10,000 sites to 100,000 sites. In some embodiments, the first set of one or more sites is from 1 site to 10,000 sites. In some embodiments, the first set of one or more sites is from 10 sites to 10,000 sites. In some embodiments, the first set of one or more sites is from 100 sites to 10,000 sites. In some embodiments, the first set of one or more sites is from 1000 sites to 10,000 sites. In some embodiments, the first set of one or more sites is from 1 site to 1000 sites. In some embodiments, the first set of one or more sites is from 10 sites to 1000 sites. In some embodiments, the first set of one or more sites is from 100 sites to 1000 sites.

In some embodiments, the set of criteria comprises at least one exclusion criterion. In some embodiments, a clinical trial eligibility criteria includes one or more inclusion criterion that must be present in order to admit a patient into a clinical trial. For example, in some embodiments, an inclusion criterion is a specific type of cancer, e.g., pancreatic cancer, meaning that a patient must have pancreatic cancer to be eligible for the clinical trial. In some embodiments, a clinical trial eligibility includes an exclusion criterion that cannot be present in order to admit a patient into a clinical trial. For example, in some embodiments, an exclusion criterion is a particular prior therapy, e.g., prior checkpoint inhibitor therapy, meaning that if the patient previously received checkpoint inhibitor therapy they are not eligible for the clinical trial. Generally, all inclusion criterion must be met in order for a patient to be eligible for a clinical trial, while the presence of a single exclusion criterion will exclude a patient from eligibility for the trial. However, labeling a particular criterion as either of inclusion or exclusion criterion is based on convention only, as any inclusion criterion can be turned into an exclusion criterion by excluding a patient lacking a certain condition and, vice versa, an exclusion criterion can be turned into an inclusion criterion by requiring the absence of a state or condition.

In some embodiments, the clinical trial is for treatment of a cancer condition and the set of criteria comprises a diagnosis of the cancer condition, administration of one or more prior therapies for the cancer condition, absence or presence of one or more biomarkers, one or more demographic parameters, or any combination thereof.

In some embodiments, the method also includes evaluating a description of the clinical trial using language pattern recognition to determine the set of criteria for the clinical trial. The language pattern recognition may include statistical pattern recognition, syntactic pattern recognition, neural pattern recognition, template-matching pattern recognition, hybrid pattern recognition, or any combination thereof. In some embodiments, the language pattern recognition includes a machine learning (ML) model trained to identify language related to the plurality of criteria in the description of the clinical trial. The ML model may be supervised (*e.g.,* linear discriminant analysis, decision trees, neural networks, or other classification algorithms) or unsupervised (*e.g.,* deep learning methods, k-means clustering, hierarchical clustering, correlation clustering, kernel principal component analysis, or other clustering algorithms). In some embodiments, the clinical trial criteria is included in a semi-structured fashion, e.g., within a participation criteria section of a submission, or draft thereof, to a regulatory body or government agency.

In some embodiments, the language pattern recognition uses a natural language processing (NLP) technique to identify language related to the plurality of criteria in the description of the clinical trial. The NLP technique makes it possible for automated interpretation of the description of the clinical trial and/or automated generation of the plurality of criteria. For instance, in some embodiments, the NLP technique includes lexical analysis, morphological analysis, syntax analysis, parsing, semantic analysis, discourse integration, pragmatic analysis, or any combination thereof. The NLP automatically parses the clinical trial protocol in determining the plurality of criteria. The parsing of a clinical trial protocol can be dependency parsing, constituency parsing, or both. Dependency parsing focuses on the relationships between words in a sentence (marking things like primary objects and predicates), whereas constituency parsing focuses on building out the parse tree using a probabilistic context-free grammar.

In some embodiments, a large language model (LLM) is used to evaluate the description of the clinical trial to identify inclusion and exclusion criteria. LLMs are transformer-based generative machine learning models trained to understand natural language. See, for example, Vaswani, Ashish, et al., "Attention is all you need," Advances in neural information processing systems 30 (2017), the content of which is incorporated herein by reference, in its entirety. For example, in some embodiments, an LLM is provided with a description of the clinical trial and prompted to list all criteria that need to be satisfied for inclusion of a patient in the clinical trial.

In some embodiments, a generative LLM can be prepared for this purpose by providing the datasets of interest to the model, so that it may interpret results. In some embodiments, the LLM is further prepared by providing it with a plurality of priming prompts including the type of queries it may handle and the types of outputs each respective query may return which teach and influence the responses the LLM will provide.

In some embodiments, the LLM is prepared by providing the model a rule set which responses must follow, e.g., through a json or excel spreadsheet which the model would match its responses which it may generate against for the best fit and fill in the blanks denoted by a named entity field in the example responses.

In some embodiments, responses generated are tested prior to returning them to the user to ensure that unit conversions and criteria are correctly matched to the respective fields while preserving the matched eligibility criteria.

As an example, the model may be provided with a template such as "potential clinical trial entrants will be matched against the following criteria: |< 25 units | of (diagnostic blood test red blood count| within |3 months| of (clinical trial start datel," where the model would be asked to fill in the blanks and further generate the search query for this. The search query may be a regular expression for text search ("") or a SQL query (""). Alternatively, instead of outputting the query results, the LLM will actually mine the data available to it and display/output the matching results.

In some embodiments, the identifying further comprises, for a respective patient in the plurality of patients, clustering medical entries from the corresponding EMR or EHR by date and assigning a corresponding earliest eligibility date for the clinical trial based on a date associated with a clustered medical entry that is an earliest indication that the respective patient met the set of criteria for the clinical trial.

In some embodiments, the first set of patient records spans the first time period and a respective expected value in the one or more expected values for the first set of one or more sites is a measure of central tendency for the number of patients identified as satisfying each respective criterion in the set of criteria in each epoch in the plurality of epochs within the first time period.

In some embodiments, the measure of central tendency is a mean of the number of patients identified as satisfying each respective criterion in the set of criteria in each epoch in the plurality of epochs within the first time period.

In some embodiments, the first set of patient records spans the first time period and a respective expected value in the one or more expected values for the first set of one or more sites is based on a trend in the number of newly eligible patients at the first set of one or more sites over a subset of the first time period.

In some embodiments, an expected value in the one or more expected values is an expected number of newly eligible patients at the first set of one or more sites over the first time period.

In some embodiments, the first expected value for enrollment of the clinical trial is a non-negative integer at least when an expected value in the one or more expected values is no less than 1.

In some embodiments, the first expected value for enrollment in the clinical trial over the second time period is predicted by forming a statistical distribution of possible values for the second time period using a factor of a respective expected value in the one or more expected values as a measure of central tendency for the statistical distribution.

In some embodiments, the statistical distribution is a Poisson distribution established using a factor of the respective expected value in the one or more expected values as the mean of the Poisson distribution.

In some embodiments, the method also includes repeating the identifying A), using B), and predicting C) for each respective additional set of one or more sites in a plurality of additional sets of one or more sites, thereby predicting the corresponding first expected value for enrollment in the clinical trial over the first time period or the second time period for each respective additional set of one or more sites.

In some embodiments, the method also includes ranking the first set of one or more sites and each respective additional set of one or more sites in the plurality of additional sets of one or more sites based on the corresponding first expected value for enrollment in the clinical trial at the corresponding site.

In some embodiments, the method also includes selecting a group of sites for the clinical trial based on comparison between the first expected value for the first set of one or more sites and the corresponding first expected value for each respective additional set of one or more sites.

In some embodiments, the plurality of additional sets of one or more sites is at least 10 sets of one or more sites.

In some embodiments, the first set of one or more sites is a single site and each respective set of one or more sites in the plurality of additional sets of one or more sites is a single respective site.

In some embodiments, the method also includes updating the set of criteria with one or more revised criteria for the clinical trial; and repeating, for each respective revised criteria in the one or more revised criteria, the determining A), using B), and predicting C), thereby predicting a corresponding first expected value for enrollment in the clinical trial over the first time period or the second time period for the updated set of criteria.

In some embodiments, the method also includes selecting a criterion for the clinical trial based on comparison between the first expected value for the set of first criteria and the corresponding first expected value for each respective updated set of criteria.

In some embodiments, the first set of one or more sites is at least 100 sites.

In some embodiments, the method also includes opening recruitment for the clinical trial.

In some embodiments, the method also includes administering a treatment in the clinical trial to a patient.

The systems and methods of the present disclosure have several advantages. For instance, the systems and methods of the present disclosure can be used to identify new trials to add to the network, and help ranking new trials based on how well current sites in the network are estimated to perform. The systems and methods of the present disclosure can also be used to help users (*e.g.*, pharmaceutical companies, hospitals, clinics) to identify sites to target for their new and existing clinical trials, better plan the duration of clinical trials, optimize resource planning between trials in their portfolio (*e.g.*, program prioritization and making go/no go decisions), improve financial forecasting by decreasing the uncertainty inherent in the recruitment portion of the clinical trial process, or any combination thereof. For instance, pharmaceutical companies can use the disclosed systems and methods to estimate recruitment in clinical sites for new trials they are developing (to choose in which sites they could run the trial), to tune eligibility criteria of new trials based on how well they fit the population of a site, or both. Hospital systems, which are often resource constrained, can use the disclosed systems and methods to help correctly allocate their clinical staff by program or trial to optimize quality and financial returns. The systems and methods of the present disclosure can further be used to predict on a time period for the number of occurrences that may appear over time in the future as well as assist monitoring for changes in the expected rate of occurrences. For instance, the systems and methods of the present disclosure can be used to predict (i) the number of patients that might arrive in a hospital, (ii) new instances of particular diagnosis in a hospital, including notifying administrators if the current rate increases beyond the predicted rate as an early alarm that something may be triggering an outbreak, (iii) instances of medication or therapy uses in a hospital, (iv) instances of supply use which may enable cost efficient supply ordering and stocking, or (v) any combination thereof.

### REFERENCES CITED AND ALTERNATIVE EMBODIMENTS

All references cited herein are incorporated herein by reference in their entirety and for all purposes to the same extent as if each individual publication or patent or patent application was specifically and individually indicated to be incorporated by reference in its entirety for all purposes.

The present invention can be implemented as a computer program product that includes a computer program mechanism embedded in a non-transitory computer-readable storage medium. For instance, the computer program product could contain instructions for operating the user interfaces disclosed herein. These program modules can be stored on a CD-ROM, DVD, magnetic disk storage product, USB key, or any other non-transitory computer readable data or program storage product.

Many modifications and variations of this invention can be made without departing from its spirit and scope, as will be apparent to those skilled in the art. The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims, along with the full scope of equivalents to which such claims are entitled.

Disclosed Items

1. A method for predicting clinical trial enrollment of a clinical trial having a set of criteria, the method comprising: at a computer system that includes one or more processors and memory:

A) determining a longitudinal trajectory of patient eligibility for the clinical trial by identifying, at each respective epoch in a plurality of epochs of a first time period, a respective set of patients for a first set of one or more sites by matching a corresponding plurality of clinical values extracted from an electronic medical record (EMR) or electronic health record (EHR) for each respective patient in a plurality of patients, that is valid for the respective epoch, to each criterion in the set of criteria to determine whether they satisfy each respective criterion in the set of criteria for the clinical trial during the respective epoch;

B) using the longitudinal trajectory of patient eligibility for the clinical trial over the first time period to extrapolate one or more expected values for the first set of one or more sites for a second time period that is other than the first time period; and

C) predicting, using the one or more expected values, a first expected value for enrollment in the clinical trial over the second time period at the first set of one or more sites.

2. The method of item 1, wherein determining the longitudinal trajectory comprises, for a respective patient in the first plurality of patient records, determining by natural language processing the corresponding plurality of clinical values that is valid for the respective epoch for the respective patient from the EMR or EHR of the respective patient.

3. The method of item 1, wherein determining the longitudinal trajectory comprises, for a respective patient in the first plurality of patient records, determining by natural language processing at least one clinical value in the corresponding plurality of clinical values that is valid for the respective epoch for the respective patient from unstructured data in the EMR or EHR of the respective patient.

4. The method of item 2 or 3, wherein the natural language processing comprises string-matching, lexical analysis, morphological analysis, syntax analysis, parsing, semantic analysis, discourse integration, pragmatic analysis, or a combination thereof.

5. The method of any one of items 1-4, wherein the determining the longitudinal trajectory queries structured clinical data in the EMR or EHR for a respective patient in the plurality of patients with a string-matching algorithm to identify a clinical value in the corresponding plurality of clinical values that is valid for an epoch in the plurality of epochs for the respective patient.

6. The method of any one of items 1-5, wherein the determining the longitudinal trajectory applies a large language model to the EMR or EHR for a respective patient in the plurality of patients to identify a clinical value in the corresponding plurality of clinical values that is valid for an epoch in the plurality of epochs for the respective patient.

7. The method of any one of items 1-6, wherein the plurality of patients is at least 100 patients.

8. The method of any one of items 1-7, wherein the set of criteria comprises at least one exclusion criterion.

9. The method of any one of items 1-8, wherein the clinical trial is for treatment of a cancer condition and the set of criteria comprises a diagnosis of the cancer condition, administration of one or more prior therapies for the cancer condition, absence or presence of one or more biomarkers, one or more demographic parameters, or any combination thereof.

10. The method of any one of items 1-9, further comprising, at the computer system:
evaluating a description of the clinical trial using language pattern recognition to determine the set of criteria for the clinical trial.

11. The method of any one of items 1-10, wherein the identifying further comprises, for a respective patient in the plurality of patients, clustering medical entries from the corresponding EMR or EHR by date and assigning a corresponding earliest eligibility date for the clinical trial based on a date associated with a clustered medical entry that is an earliest indication that the respective patient met the set of criteria for the clinical trial.

12. The method of any one of items 1-11, wherein the first set of patient records spans the first time period and a respective expected value in the one or more expected values for the first set of one or more sites is a measure of central tendency for the number of patients identified as satisfying each respective criterion in the set of criteria in each epoch in the plurality of epochs within the first time period.

13. The method of item 12, wherein the measure of central tendency is a mean of the number of patients identified as satisfying each respective criterion in the set of criteria in each epoch in the plurality of epochs within the first time period.

14. The method of any one of items 1-11, wherein the first set of patient records spans the first time period and a respective expected value in the one or more expected values for the first set of one or more sites is based on a trend in the number of newly eligible patients at the first set of one or more sites over a subset of the first time period.

15. The method of any one of items 1-14, wherein an expected value in the one or more expected values is an expected number of newly eligible patients at the first set of one or more sites over the first time period.

16. The method of any one of items 1-15, wherein the first expected value for enrollment of the clinical trial is a non-negative integer at least when an expected value in the one or more expected values is no less than 1.

17. The method of any one of items 1-16, wherein the first expected value for enrollment in the clinical trial over the second time period is predicted by forming a statistical distribution of possible values for the second time period using a factor of a respective expected value in the one or more expected values as a measure of central tendency for the statistical distribution.

18. The method of item 17, wherein the statistical distribution is a Poisson distribution established using a factor of the respective expected value in the one or more expected values as the mean of the Poisson distribution.

19. The method of any one of items 1-17, further comprising repeating the identifying A), using B), and predicting C) for each respective additional set of one or more sites in a plurality of additional sets of one or more sites, thereby predicting the corresponding first expected value for enrollment in the clinical trial over the first time period or the second time period for each respective additional set of one or more sites.

20. The method of item 19, further comprising ranking the first set of one or more sites and each respective additional set of one or more sites in the plurality of additional sets of one or more sites based on the corresponding first expected value for enrollment in the clinical trial at the corresponding site.

21. The method of item 19 or 20, further comprising selecting a group of sites for the clinical trial based on comparison between the first expected value for the first set of one or more sites and the corresponding first expected value for each respective additional set of one or more sites.

22. The method of any one of items 19-21, wherein the plurality of additional sets of one or more sites is at least 10 sets of one or more sites.

23. The method of any one of items 19-22, wherein the first set of one or more sites is a single site and each respective set of one or more sites in the plurality of additional sets of one or more sites is a single respective site.

24. The method of any one of items 1-23, further comprising:

updating the set of criteria with one or more revised criteria for the clinical trial; and
repeating, for each respective revised criteria in the one or more revised criteria, the determining A), using B), and predicting C), thereby predicting a corresponding first expected value for enrollment in the clinical trial over the first time period or the second time period for the updated set of criteria.

25. The method of item 24, further comprising selecting a criterion for the clinical trial based on comparison between the first expected value for the set of first criteria and the corresponding first expected value for each respective updated set of criteria.

26. The method of any one of items 1-22 and 24-25, wherein the first set of one or more sites is at least 100 sites.

27. The method of any one of items 1-26, further comprising opening recruitment for the clinical trial.

28. The method of any one or items 1-27, further comprising administering a treatment in the clinical trial to a patient.

29. A computer system comprising:

one or more processors; and
a non-transitory computer-readable medium including computer-executable instructions that, when executed by the one or more processors, cause the processors to perform the method according to any one of items 1-28.

30. A non-transitory computer-readable storage medium having stored thereon program code instructions that, when executed by a processor, cause the processor to perform the method according to any one of items 1-28.

**Claims**

1. A method for predicting clinical trial enrollment of a clinical trial having a set of criteria, the method comprising:
   at a computer system that includes one or more processors and memory:

   A) determining a longitudinal trajectory of patient eligibility for the clinical trial by identifying, at each respective epoch in a plurality of epochs of a first time period,
   a respective set of patients for a first set of one or more sites by matching a corresponding plurality of clinical values extracted from an electronic medical record (EMR) or electronic health record (EHR) for each respective patient in a plurality of patients, that is valid for the respective epoch, to each criterion in the set of criteria to determine whether the respective patient satisfies each respective criterion in the set of criteria for the clinical trial during the respective epoch;

B) using the longitudinal trajectory of patient eligibility for the clinical trial over the first time period to extrapolate one or more expected values for the first set of one or more sites for a second time period that is other than the first time period; and

C) predicting, using the one or more expected values, a first expected value for enrollment in the clinical trial over the second time period at the first set of one or more sites.

2. The method of claim 1, wherein determining the longitudinal trajectory comprises, for a respective patient in the first plurality of patient records:

determining by natural language processing the corresponding plurality of clinical values that is valid for the respective epoch for the respective patient from the EMR or EHR of the respective patient, or

determining by natural language processing at least one clinical value in the corresponding plurality of clinical values that is valid for the respective epoch for the respective patient from unstructured data in the EMR or EHR of the respective patient.

3. The method of claim 1 or 2, wherein the determining the longitudinal trajectory:

queries structured clinical data in the EMR or EHR for a respective patient in the plurality of patients with a string-matching algorithm to identify a clinical value in the corresponding plurality of clinical values that is valid for an epoch in the plurality of epochs for the respective patient, or

applies a large language model to the EMR or EHR for a respective patient in the plurality of patients to identify a clinical value in the corresponding plurality of clinical values that is valid for an epoch in the plurality of epochs for the respective patient.

4. The method of any one of claims 1-3, wherein the plurality of patients is at least 100 patients.

5. The method of any one of claims 1-4, wherein the set of criteria comprises at least one exclusion criterion.

6. The method of any one of claims 1-5, wherein the clinical trial is for treatment of a cancer condition and the set of criteria comprises a diagnosis of the cancer condition, administration of one or more prior therapies for the cancer condition, absence or presence of one or more biomarkers, one or more demographic parameters, or any combination thereof.

7. The method of any one of claims 1-6, wherein the identifying further comprises, for a respective patient in the plurality of patients, clustering medical entries from the corresponding EMR or EHR by date and assigning a corresponding earliest eligibility date for the clinical trial based on a date associated with a clustered medical entry that is an earliest indication that the respective patient met the set of criteria for the clinical trial.

8. The method of any one of claims 1-7, wherein the first set of patient records spans the first time period and a respective expected value in the one or more expected values for the first set of one or more sites is a measure of central tendency for the number of patients identified as satisfying each respective criterion in the set of criteria in each epoch in the plurality of epochs within the first time period, wherein optionally the measure of central tendency is a mean of the number of patients identified as satisfying each respective criterion in the set of criteria in each epoch in the plurality of epochs within the first time period.

9. The method of any one of claims 1-8, wherein the first set of patient records spans the first time period and a respective expected value in the one or more expected values for the first set of one or more sites is based on a trend in the number of newly eligible patients at the first set of one or more sites over a subset of the first time period.

10. The method of any one of claims 1-9, wherein an expected value in the one or more expected values is an expected number of newly eligible patients at the first set of one or more sites over the first time period.

11. The method of any one of claims 1-10, wherein

the first expected value for enrollment of the clinical trial is a non-negative integer at least when an expected value in the one or more expected values is no less than 1, or
the first expected value for enrollment in the clinical trial over the second time period is predicted by forming a statistical distribution of possible values for the second time period using a factor of a respective expected value in the one or more expected values as a measure of central tendency for the statistical distribution, wherein optionally the statistical distribution is a Poisson distribution established using a factor of the respective expected value in the one or more expected values as the mean of the Poisson distribution.

12. The method of any one of claims 1-11, further comprising repeating the identifying A), using B), and predicting C) for each respective additional set of one or more sites in a plurality of additional sets of one or more sites, thereby predicting the corresponding first expected value for enrollment in the clinical trial over the first time period or the second time period for each respective additional set of one or more sites, and

optionally ranking the first set of one or more sites and each respective additional set of one or more sites in the plurality of additional sets of one or more sites based on the corresponding first expected value for enrollment in the clinical trial at the corresponding site, or
optionally selecting a group of sites for the clinical trial based on comparison between the first expected value for the first set of one or more sites and the corresponding first expected value for each respective additional set of one or more sites.

13. The method of any one of claims 1-12, further comprising:

updating the set of criteria with one or more revised criteria for the clinical trial; and
repeating, for each respective revised criteria in the one or more revised criteria, the determining A), using B), and predicting C), thereby predicting a corresponding first expected value for enrollment in the clinical trial over the first time period or the second time period for the updated set of criteria, and optionally
selecting a criterion for the clinical trial based on comparison between the first expected value for the set of first criteria and the corresponding first expected value for each respective updated set of criteria.

14. A computer system comprising:

one or more processors; and
a non-transitory computer-readable medium including computer-executable instructions that, when executed by the one or more processors, cause the processors to perform the method according to any one of items 1-13.

15. A non-transitory computer-readable storage medium having stored thereon program code instructions that, when executed by a processor, cause the processor to perform the method according to any one of items 1-13.

**100**

| | |
|---|---|
| Operating system | 34 |
| Input output module | 64 |
| Patient records | 36 |
|     Clinical data (structured and/or unstructured) | 38 |
| Language pattern recognition module | 40 |
| Expressions | 42 |
| Aggregation module | 52 |
| Interpretation module | 54 |
| Clustering module | 56 |
| Training module | 58 |
|     Labels | 60 |
|     Training datasets | 62 |
|     ⋮ | |
| ⋮ | |

92

90

Controller
88

12

CPU
59

79

Power
supply

78

User interface
Display
Keyboard

82
80

84

Network
interface

Fig. 1

**Step #1** - Process [Trial Eligibility Criteria] for [patient matching] use

**Step #2** - Use trial [inclusion/exclusion] criteria as "search query" against Clinical Mart

Clinical Mart

Expert DEID

**Step #3.1** - Aggregate "search query" output - by site, trial snapshot month to extrapolate [expected values]

| Site A | 01-01-2021 | 1 | NCT123 |
|--------|------------|---|--------|
| Site A | 02-01-2021 | 3 | NCT123 |
| Site B | 01-01-2021 | 2 | NCT123 |
| Site C | 05-01-2021 | 0 | NCT123 |
| Site D | 01-01-2021 | 6 | NCT123 |

**Step #3.2** - Extrapolate [expected values]

| Site A | NCT123 | 0.43 |
|--------|--------|------|
| Site B | NCT123 | 0.68 |
| Site C | NCT123 | 0.01 |
| Site D | NCT123 | 1.23 |

**Step #4** - Make predictions for provided trial(s)

| Site A | NCT123 | 1 / month | 12 / year |
|--------|--------|-----------|-----------|
| Site B | NCT123 | 2 / month | 24 / year |
| Site C | NCT123 | 1 / month | 12 / year |
| Site D | NCT123 | 1.25 / month | 15 / year |
| Site E | NCT123 | 0.5 / month | 6 / year |

**Step #5** - Share results/output with stakeholders

Internal Snapshot

External Report

Fig. 2

300

*(302)* Evaluating a description of a clinical trial using language pattern recognition to determine a plurality of criteria for a clinical trial.

*(304)* Identifying, for each respective site in a plurality of sites, a corresponding set of patient records from a corresponding plurality of patient records for the respective site, wherein (i) each respective patient record in the corresponding plurality of patient records comprises corresponding data from an electronic medical record (EMR) or electronic health record (EHR) for a respective patient in a corresponding plurality of patients and (ii) the identifying comprises filtering the corresponding plurality of patient records to identify the corresponding set of patient records in the corresponding plurality of patient records that each includes, for each respective criterion in the plurality of criteria for the clinical trial, an expression indicating satisfaction of the respective criterion in the corresponding data.

*(306)* Generating, for each respective site in the plurality of sites, a corresponding longitudinal trajectory of patient eligibility for the clinical trial over a time period at the respective site based on the corresponding set of patient records for the respective site.

*(308)* Creating, for each respective site in the plurality of sites, a corresponding set of snapshots based on the corresponding set of patient records, wherein each respective snapshot in the set of snapshots is for a corresponding patient in a set of patients from the corresponding plurality of patients, and comprises a corresponding set of indicators each indicating eligibility of the corresponding patient for the clinical trial at a corresponding time point in the plurality of time points.

*(310)* Identifying, based on the set of clinical events and the corresponding set of patient records, a corresponding set of instances for each corresponding patient in the set of patients, wherein each instance in the corresponding set of instances represents an occurrence of a clinical event in the set of clinical events for the corresponding patient.

(A)

Fig. 3A

A

**(306 continued)**

**(308 continued)**

**(312)** Determining, for each corresponding patient in the set of patients, eligibility of the corresponding patient for the clinical trial at any one of the plurality of time points based on the corresponding set of instances for the corresponding patient, thereby creating the corresponding set of snapshots.

**(314)** Aggregating, for each respective site in the plurality of sites, the corresponding set of snapshots, thereby generating the corresponding longitudinal trajectory of patient eligibility for the clinical trial over the time period at the respective site.

**(316)** Predicting, for each respective site in the plurality of sites, a corresponding set of expected values for enrollment in the clinical trial at the respective site based on the corresponding longitudinal trajectory of patient eligibility for the clinical trial, wherein each expected value in the corresponding set of expected values is predicted for a target time period in a set of target time periods.

**(318)** Determining a plurality of possible values for the number of eligible patients based on the plurality of criteria for the clinical trial.

**(320)** Determining, for each respective target time period in the set of target time periods, a probability of getting each respective passible value in the plurality of possible values based on the corresponding longitudinal trajectory of patient eligibility for the clinical trial, thereby producing a corresponding plurality of probabilities for the respective target time period.

B

Fig. 3B

B

**(316 continued)**

**(322)** Multiplying, for each respective target time period in the set of target time periods, the plurality of possible values with the corresponding plurality of probabilities, thereby obtaining the corresponding set of expected values for enrollment in the clinical trial at the respective site.

**(324)** Estimating, using longitudinal data analysis, a trend based on the longitudinal trajectory of patient eligibility for the clinical trial over the time period at the respective site; and

**(326)** Extrapolating the trend to obtain an expected value in the corresponding set of expected values for enrollment in the clinical trial at the respective site.

**(328)** Repeating, for each respective additional clinical trial in a set of additional clinical trials, the identifying, generating and predicting steps to obtain a corresponding set of expected values for enrollment in the respective additional clinical trial at each respective site in the plurality of site.

**(330)** Ranking each respective site in the plurality of sites based on the corresponding set of expected values for enrollment in the clinical trial at the respective site.

**(332)** Creating a presentation.

Fig. 3C

Fig. 4

EP 4 394 784 A1

## STANDARD CLINICAL DATA ELEMENTS

**PATIENT INFO**

-Gender
-Age at diagnosis
-Race/ethnicity
-Smoking status
-Relevant comorbidities

**DIAGNOSIS**

-Cancer site
-Date of diagnosis
-Histology
-Grade
-T,N,M, Stage

**TREATMENT**

-Medication (type and dates)
-Medication dosage and cycles
-Surgery & other oncology procedures
(type, dates)
-Radiation (type and dates)
-Radiation site and fractions

**OUTCOMES**

-Response to therapy
(RECIST if available)
-Dates of outcomes
-Adverse events
-Imaging (type, date,
tumor response)
-Date of last follow up
-Date of death

**MOLECULAR DATA**

-Past molecular testing
reports (NGS or small panel)
-Key subtype-specific
genomic indications, for
example:
EGFR, ALK, ROS, KRAS,
MSI, TMB, PD-L1, others

**ASSESSMENTS**

Performance Status (ECOG / Karnofsky
and dates)

**Example labs captured from EMR
data:** LDH, Creatinine, B2M, Albumin,
Calcium, Hemoglobin, IgA, IgG, IgM, IgD,
IgE, Serum Free Kappa, Serum Free
Lambda, Bence Jones Protein, Serum M-
Spike, Serum Immunofixation, Urine
Immunofixation, % clonal plasma cells

## MOLECULAR DATA ELEMENTS

**TUMOR / NORMAL DNA SEQUENCING DATA:**

o SNVs, CNVs, Indels, and fusions for select genes

**WHOLE TRANSCRIPTOME RNA SEQUENCING DATA:**

o RNA expression and fusions

**IHC IMAGING DATA:**

o MSI, TMB, MMR, and PD-L1 IHC

**EMERGING IMMUNOTHERAPY DATA FROM DNA/RNA**

o Immune infiltration, immune biomarker expression,
neo-antigen prediction, and HLA typing.

Fig. 5

EP 4 394 784 A1

Site-1

$\{$ Patient-1 ... Patient-K1 $\}$ ~420-1

$\{$ Patient-1 Patient-2 Patient-3 ... $\}$ ~440-1

$\{$ Patient Record $\}$ ~430-1

$\{$ Snapshot-1 ({Indicator}-1) Snapshot-2 ({Indicator}-2) Snapshot-3 ({Indicator}-3) ... $\}$ ~610-1

Site-2

$\{$ Patient-1 ... Patient-K2 $\}$ ~420-2

$\{$ Patient $\}$ ~440-2

$\{$ Patient Record $\}$ ~430-2

$\{$ Snapshot $\}$ ~610-2

■  ■  ■  ■  ■  ■

Fig. 6

| Trial Name | DEIDENTIFIED SITE | |
|---|---|---|
| Calithera KEAPSAKE | 8170 | 13 |
| | 8171 | 10 |
| | 8167 | 7 |
| | 7973 | 6 |
| | 7340 | 5 |
| | 67118 | 4 |
| | 8186 | 3 |
| | 8194 | 3 |
| | 8185 | 2 |
| | 7977 | 2 |
| | 65128 | 1 |
| | 8165 | 1 |
| | 6781 | 1 |
| | 6659 | 1 |
| | 8180 | 1 |
| | 7979 | 1 |
| | 8177 | 1 |
| | 7880 | 1 |
| | 8692 | 1 |
| | 8187 | 1 |
| | 8173 | 1 |
| | 8163 | 1 |
| | 7981 | 0 |
| | 8159 | 0 |
| | 7752 | 0 |
| | 7349 | 0 |
| | 8363 | 0 |
| | 8055 | 0 |

Fig. 7A

Fig. 7B

**800**

(802) Evaluating a description of each respective clinical trial in a set of clinical trials using language pattern recognition to determine a corresponding plurality of criteria for the respective clinical trial.

(804) Obtaining, for each respective site in a plurality of sites, a corresponding plurality of patient records for the respective site, wherein each respective patient record in the corresponding plurality of patient records comprises corresponding data from an electronic medical record (EMR) or electronic health record (EHR) for a respective patient in a corresponding plurality of patients.

(806) Identifying, for each respective site in the plurality of sites and for each respective clinical trial in a set of clinical trials, a corresponding set of patient records from the corresponding plurality of patient records for the respective site, wherein (i) a corresponding plurality of criteria is extracted from the respective clinical trial, and (ii) the identifying comprises filtering the corresponding plurality of patient records such that each of the corresponding set of patient records includes, for each respective criterion in the corresponding plurality of criteria for the respective clinical trial, an expression indicating satisfaction of the respective criterion in the corresponding data.

(808) Generating, for each respective site in the plurality of sites and for each respective clinical trial in the set of clinical trials, a corresponding longitudinal trajectory of patient eligibility for the respective clinical trial over a corresponding time period at the respective site based on the corresponding set of patient records.

(A)

Fig. 8A

(A)

**(810)** Predicting, for each respective site in the plurality of sites and for each respective clinical trial in the set of clinical trials, a corresponding set of expected values for enrollment in the respective clinical trial at the respective site based on the corresponding longitudinal trajectory of patient eligibility for the respective clinical trial, wherein each expected value in the corresponding set of expected values is predicted for a target time period in a corresponding set of target time periods.

**(812)** Ranking each respective site in the plurality of sites based on the corresponding set of expected values for enrollment in the respective clinical trial at the respective site.

**(814)** Creating a presentation.

## Fig. 8B

**(902)** Evaluate a description of a clinical trial using language pattern recognition to determine a first criteria for the clinical trial.

**(904)** Identify a first set of patient records from first a plurality of patient records for a first set of one or more sites, wherein each respective patient record in the corresponding plurality of patient records comprises corresponding data from an electronic medical record (EMR) or electronic health record (EHR) for a respective patient in a plurality of patients, that satisfies each respective criterion in a first criteria for a clinical trial.

**(906)** Determine by natural language processing, for a respective patient record in the first plurality of patient records, whether the corresponding data includes, for each respective criterion in the plurality of criterion, a corresponding expression that satisfies the respective criterion.

**(908)** The corresponding data comprises unstructured clinical data from the electronic medical record (EMR) or electronic health record (EHR) and identifying the first set of patient records comprises, for a respective criterion in the plurality of criterion, using natural language processing to determine whether the unstructured clinical data includes an expression satisfying the respective criterion.

**(910)** The natural language processing comprises string-matching, lexical analysis, morphological analysis, syntax analysis, parsing, semantic analysis, discourse integration, pragmatic analysis, or a combination thereof.

Ⓐ

Fig. 9A

(A)

**(904 continued)**

**(912)** The corresponding data comprises structured clinical data from the electronic medical record (EMR) or electronic health record (EHR) and identifying the first set of patient records comprises, for a respective criterion in the plurality of criterion, using a string-matching algorithm to determine whether the structured clinical data includes an expression satisfying the respective criterion.

**(914)** using a large language model to determine, for a respective patient record in the first plurality of patient records, whether the corresponding data indicates that the respective patient meets the first criteria for the clinical trial.

**(916)** The first plurality of patient records is at least 100 patient records.

**(918)** The first criteria comprises at least one exclusion criterion that is satisfied.

**(920)** The clinical trial is for treatment of a cancer condition and the first criteria comprises a diagnosis of the cancer condition, administration of one or more prior therapies for the cancer condition, one or more biomarkers, one or more demographic parameters, or any combination thereof.

**(922)** The first set of one or more sites is at least 100 sites.

(B)

Fig. 9B

B

(923) Determine a first longitudinal trajectory of patient eligibility for the clinical trial over a first time period at the first set of one or more sites based on the first set of patient records.

(924) Cluster, for a respective patient record in the plurality of patient records, medical entries in the corresponding data based on dates and assigning a corresponding earliest eligibility date for the clinical trial based on a date associated with a clustered medical entry that is an earliest indication that the respective patient met the first criteria for the clinical trial.

(926) the first set of patient records spans a first time period and the longitudinal trajectory is a measure of central tendency for the number of patient records identified in each of a plurality of time subdivisions within the first time period.

(928) The measure of central tendency is a mean of the number of patient records identified in each of a plurality of time subdivisions within the first time period.

(930) The first set of patient records spans a first time period and the logitudinal trajectory is based on a trend in the number of newly eligible patients at the first set of one or more sites over a second time period that is shorter than the first time period.

C

Fig. 9C

C

(931) Predict a first expected value for enrollment in the clinical trial over a first time period at the first set of one or more sites using a statistical distribution formed on the basis of the first longitudinal trajectory of patient eligibility for the clinical trial.

(932) The first expected value is an expected number of newly eligible patients at the first set of one or more sites over the first time period, determined using the statistical distribution.

(934) The first expected value is an expected number of new enrollments at the first set of one or more sites over the first time period, determined as a weighted value of an expected number of newly eligible patients at the first set of one or more sites over the first time period based on the statistical distribution.

(936) The first expected value for enrollment of in the clinical trial is a non-negative integer at least when the expected value is no less than 1.

(938) The statistical distribution is a Poisson distribution formed using a factor of the first longitudinal trajectory as the mean of the distribution.

(940) Determine a second expected value for enrollment in the clinical trial over a second time period at the first set of one or more sites using a statistical distribution formed on the basis of the first longitudinal trajectory of patient eligibility for the clinical trial.

D          E          F          G

Fig. 9D

D

**(941)** Repeat the identifying, determining, and predicting for each respective additional set of one or more sites in a plurality of additional sets of one or more sites, thereby predicting corresponding first expected value for enrollment in the clinical trial over the first time period for each respective additional set of one or more sites.

**(942)** The plurality of additional sets of one or more sites is at least 10 sets of one or more sites.

**(944)** The first set of one or more sites is a single site and each respective set of one or more sites in the plurality of additional sets of one or more sites is a single respective site.

**(946)** Rank the first set of one or more sites and each respective set of one or more sites in the plurality of additional sets of one or more sites based on the corresponding first expected value for enrollment in the clinical trial at the corresponding site.

**(948)** Select a group of sites for the clinical trial based on comparison between the first expected value for the first set of one or more sites and the corresponding first expected value for each respective additional set of one or more sites.

F          G

Fig. 9E

(E)

(949) Generate one or more revised criteria for the clinical trial.

(950) Repeat, for each respective revised criteria in the one or more revised criteria, the identifying A), determining B), and predicting C), thereby predicting a corresponding first expected value for enrollment in the clinical trial over the first time period for the respective revised criteria.

(952) Select a criteria for the clinical trial based on comparison between the first expected value for the first criteria and the corresponding first expected value for each respective revised criteria.

(F)   (G)

(F)

(953) Open recruitment for the clinical trial.

(G)

(955) Administer a treatment in the clinal trial to a patient.

Fig. 9F

Europäisches
Patentamt

European
Patent Office

Office européen
des brevets

# EUROPEAN SEARCH REPORT

Application Number

EP 23 22 0596

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/182470 A1 (KALATHIL RAVI K [US]) 28 June 2018 (2018-06-28) * abstract; figures 1a,2 * * paragraph [0022] – paragraph [0043] * * paragraph [0119] – paragraph [0125] * * paragraph [0052] – paragraph [0055] * ----- | 1-15 | INV. G16H10/20 G16H10/60 G16H50/20 G16H50/30 G16H50/70 |
| X | WO 2016/203457 A1 (KONINKLIJKE PHILIPS NV [NL]) 22 December 2016 (2016-12-22) * abstract; figure 4 * * page 1 – page 5 * * page 7 * ----- | 1-15 | |
| A | WO 2019/022779 A1 (GOOGLE LLC [US]) 31 January 2019 (2019-01-31) * the whole document * ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 15 April 2024 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 22 0596

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2024

| Patent document cited in search report | | Publication date | Patent family member(s) | | Publication date |
|---|---|---|---|---|---|
| US 2018182470 | A1 | 28-06-2018 | US  2015161336 | A1 | 11-06-2015 |
| | | | US  2018182470 | A1 | 28-06-2018 |
| WO 2016203457 | A1 | 22-12-2016 | EP  3311317 | A1 | 25-04-2018 |
| | | | US  2018301205 | A1 | 18-10-2018 |
| | | | WO  2016203457 | A1 | 22-12-2016 |
| WO 2019022779 | A1 | 31-01-2019 | CN  110691548 | A | 14-01-2020 |
| | | | EP  3634204 | A1 | 15-04-2020 |
| | | | JP  7030853 | B2 | 07-03-2022 |
| | | | JP  2020529057 | A | 01-10-2020 |
| | | | KR  20200003407 | A | 09-01-2020 |
| | | | US  2019034589 | A1 | 31-01-2019 |
| | | | US  2019034590 | A1 | 31-01-2019 |
| | | | US  2019034591 | A1 | 31-01-2019 |
| | | | WO  2019022779 | A1 | 31-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63478040
- US 63420466
- US 8843482 B

**Non-patent literature cited in the description**

- **FOGEL DB.** *Contemporary Clinical Trials Communications,* 2018, vol. 11, 156-64
- **LAGOR et al.** *Journal of Clinical Oncology,* 2022, vol. 40 (16), 1558
- **BIEGANEK C et al.** *PLoS ONE,* 2022, vol. 17 (2), e0263193
- **SIMILARLY, LIU J. et al.** A Machine Learning Approach for Recruitment Prediction in Clinical Trial Design. *arXiv:2111.07407,* 2021
- **DUDA ; HART.** Pattern Classification and Scene Analysis. John Wiley & Sons, Inc, 1973, 211-256
- **MITCHELL.** Machine Learning. McGraw-Hill, 1997
- **VASWANI ; ASHISH et al.** Attention is all you need. *Advances in neural information processing systems,* 2017, vol. 30